# EUROPEAN PATENT APPLICATION

(11) **EP 1 197 557 A1**
(43) Date of publication of application: **17.04.2002**
(21) Application number: 00940872.5
(22) Date of filing: 28.06.2000
(51) Int. Cl.: C12N 15/55, C12N 1/21, C12N 9/14

(54) **GENE ENCODING CYCLIC LIPOPEPTIDE ACYLASE AND EXPRESSION OF THE SAME**

(30) Priority: 02.07.1999 JP 18964499
(71) Applicant: FUJISAWA PHARMACEUTICAL CO., LTD., Osaka-shi Osaka 541-8514 (JP)
(72) Inventor: SHIBATA, Takashi, Ibaraki 305-0051 (JP); NOGUCHI, Yuji, Ama-gun, Aichi 490-1114 (JP); YAMASHITA, Michio, Tsukuba-shi, Ibaraki 305-0044 (JP)
(74) Representative: Gille Hrabal Struck Neidlein Prop Roos
(86) International application number: JP0004285
(87) International publication number: WO0102585

(57) **Abstract**

The present invention relates to determination of the nucleotide sequence of coding region of cyclic lipopeptide acylase, determination of the full-length amino acid sequence of the acylase, an expression vector containing a gene encoding the enzyme, and a method for producing cyclic lipopeptide acylase by expression of the expression vector in a host cell. According to the present invention, the use of the transformant having the same level of activity as does an acylase obtained by culturing a conventional cyclic lipopeptide acylase producing bacteria, which is obtained by genetic engineering has made it possible to shorten the time necessary for culture (time necessary for producing cyclic lipopeptide acylase).

## Description

### Technical Field

The present invention relates to an enzyme that deacylates an acyl side chain of a cyclic lipopeptide substance (hereinafter to be also referred to as cyclic lipopeptide acylase), a gene encoding same, a method of producing cyclic lipopeptide acylase by genetic engineering using the gene and a method of deacylating an acyl side chain of a cyclic lipopeptide substance.

### Background Art

As an enzyme deacylating an acyl side chain of cyclic lipopeptide substances, such as FR901379 substance and analogs thereof, there has been reported enzymes produced by the bacteria belonging to the genus *Streptomyces* (e.g., *Streptomyces anulatus* No. 4811 strain, *Streptomyces anulatus* No. 8703 strain, *Streptomyces sp.* No. 6907 strain) (WO97/32975). WO97/47738 further reports enzymes produced by *Oidiodendron tenuissimum* IFO 6798 strain, *Oidiodendron echinulatum* IFO 31963 strain, *Oidiodendron truncatum* IFO 9951 strain, *Oidiodendron truncatum* IFO 31812 strain, *Oidiodendron sp.* No. 30084 strain and *Verticillium sp.* No. 30085 strain.

A large scale production and shortened production time of these enzymes have been desired.

### Disclosure Of The Invention

The present invention aims at harvesting cyclic lipopeptide acylase more efficiently. More particularly, the present invention aims at determining the amino acid sequence of the cyclic lipopeptide acylase, determining a gene that encodes the same, and providing a method for producing the enzyme by genetic engineering using the gene and a method for deacylating an acyl side chain of a cyclic lipopeptide substance.

The present inventors have conducted intensive studies in an attempt to achieve the above-mentioned objects and succeeded in cloning the coding region of cyclic lipopeptide acylase, which resulted in the completion of the present invention. The present inventors have further introduced an expression vector containing the DNA into a host cell to allow expression of the activity of the cyclic lipopeptide acylase.

Accordingly, the present invention provides the following.
[1] A gene encoding cyclic lipopeptide acylase, which contains the entirety or a part of the following (a), (b) or (c):
   (a) a DNA consisting of the nucleotide sequence depicted in SEQ ID No. 1
   (b) a DNA capable of hybridizing with the DNA of the above-mentioned (a) under stringent conditions
   (c) a DNA having at least (1) 60% identity, (2) 70% identity, (3) 80% identity, (4) 90% identity or (5) 95% identity with the nucleotide sequence depicted in SEQ ID No. 1.
[2] A gene encoding a protein of the following (a) or (b) or a part thereof:
   (a) a protein consisting of the amino acid sequence depicted in SEQ ID No. 2
   (b) a protein having an amino acid sequence involving deletion, substitution or addition of one to several amino acid(s) in the amino acid sequence (a), which protein has a cyclic lipopeptide acylase activity.
[3] A recombinant vector containing the gene of the above-mentioned [1] or [2].
[4] An expression vector functionally containing the gene of the above-mentioned [1] or [2].
[5] A transformant obtained by transforming a host cell with the vector of the above-mentioned [3] or [4].
[6] A method of producing cyclic lipopeptide acylase, which comprises
   culturing a host cell transformed with the expression vector of the above-mentioned [4], and
   harvesting, from the obtained culture, cyclic lipopeptide acylase capable of catalyzing a reaction to deacylate a side chain acylamino group of a cyclic lipopeptide substance into an amino group.
[7] A cyclic lipopeptide acylase produced by the production method of the above-mentioned [6].
[8] A gene encoding cyclic lipopeptide acylase, which contains the entirety or a part of the following (a), (b) or (c):
   (a) a DNA consisting of a nucleotide sequence shown by nucleotide No. 1065 to 3359 in the nucleotide sequence depicted in SEQ ID No. 1
   (b) a DNA capable of hybridizing with the DNA of the above-mentioned (a) under stringent conditions
   (c) a DNA having at least (1) 60% identity, (2) 70% identity, (3) 80% identity, (4) 90% identity or (5) 95% identity with the nucleotide sequence shown by nucleotide No. 1065 to 3359 in the nucleotide sequence depicted in SEQ ID No. 1.
[9] A gene encoding a protein of the following (a) or (b):
   (a) a protein consisting of amino acid number from -1 or 1 to 765 in the amino acid sequence depicted in SEQ ID No. 2
   (b) a protein having an amino acid sequence involving deletion, substitution or addition of one to several amino acid(s) in the amino acid sequence (a), which protein has a cyclic lipopeptide acylase activity.
[10] A recombinant vector containing the gene of the above-mentioned [8] or [9].
[11] An expression vector functionally containing the gene of the above-mentioned [8] or [9].
[12] A transformant obtained by transforming a host cell with a vector of the above-mentioned [10] or [11].
[13] A method of producing cyclic lipopeptide acylase, which comprises
   culturing a host cell transformed with the expression vector of the above-mentioned [11], and
   harvesting, from the obtained culture, cyclic lipopeptide acylase capable of catalyzing a reaction to deacylate a side chain acylamino group of a cyclic lipopeptide substance into an amino group.
[14] A cyclic lipopeptide acylase produced by the production method of the above-mentioned [13].
[15] A cyclic lipopeptide acylase encoded by a DNA consisting of a nucleotide sequence shown by nucleotide No. 1065 to 3359 in the nucleotide sequence depicted in SEQ ID No. 1.
[16] A cyclic lipopeptide acylase which is encoded by a DNA having at least (1) 60% identity, (2) 70% identity, (3) 80% identity, (4) 90% identity or (5) 95% identity with the nucleotide sequence shown by nucleotide No. 1065 to 3359 in the nucleotide sequence depicted in SEQ ID No. 1.
[17] A protein of the following (a) or (b):
   (a) a protein consisting of the amino acid number from -1 or 1 to 200 in the amino acid sequence depicted in SEQ ID No. 2
   (b) a protein having an amino acid sequence involving deletion, substitution or addition of one to several amino acid(s) in the amino acid sequence (a), which protein forms a complex with the protein of the following [18] to show a cyclic lipopeptide acylase activity.
[18] A protein of the following (c) or (d):
   (c) a protein consisting of the amino acid No. 201 to 765 in the amino acid sequence depicted in SEQ ID No. 2
   (d) a protein having an amino acid sequence involving deletion, substitution or addition of one to several amino acid(s) in the above-mentioned amino acid sequence (c), which protein forms a complex with the protein of the above-mentioned [17] to show a cyclic lipopeptide acylase activity.
[19] A DNA encoding the protein of the above-mentioned [17].
[20] A DNA encoding the protein of the above-mentioned [18].
[21] A recombinant vector containing at least one of the above-mentioned [19] and [20].
[22] An expression vector containing at least one of the above-mentioned [19] and [20].
[23] A transformant obtained by transforming a host cell with the vector of the above-mentioned [21] or [22].
[24] A method of producing cyclic lipopeptide acylase, which comprises
   culturing a host cell transformed with the expression vector of the above-mentioned [22], and
   harvesting, from the obtained culture, cyclic lipopeptide acylase capable of catalyzing a reaction to deacylate a side chain acylamino group of a cyclic lipopeptide substance into an amino group.
[25] A cyclic lipopeptide acylase produced by the production method of the above-mentioned [24].
[26] A method for deacylating a side chain acylamino group of a cyclic lipopeptide substance into ah amino group, which method comprising culturing a host cell transformed with the expression vector of the above-mentioned [4], [11] or [22], and bringing the cyclic lipopeptide substance into contact with the obtained culture or a treated product thereof.

### Brief Description Of The Drawings

Fig. 1 shows a determined N-terminal amino acid sequence of part of the cyclic lipopeptide acylase small subunit.
Fig. 2 shows a determined N-terminal amino acid sequence of part of the cyclic lipopeptide acylase large subunit.
Fig. 3 shows a gene map of *Streptomyces lividans* 1326/pIJ702-SB.

### Detailed Description Of The Invention

In the present invention, cyclic lipopeptide acylase is an enzyme that deacylates an acyl side chain of, for example, FR901379 substance, an analog thereof and a relative, such as echinocandin B.

The cyclic lipopeptide acylase of the present invention consists of two, large and small, subunits, and each subunit has the following characteristics. Each subunit forms a complex, and shows the cyclic lipopeptide acylase activity.

### Large subunit:

(i) Molecular weight: about 61 kDa (SDS-PAGE)
(ii) Amino acid analysis:
   The N-terminal amino acid sequence is Ser-Asn-Ala-Val-Ala-Phe-Asp-Gly-Ser-Thr-Thr-Val-Asn-Gly-Arg-Gly-Leu-Leu-Leu-Gly (SEQ ID No. 3) or an amino acid sequence involving deletion, substitution or addition of one to several amino acid(s) in said amino acid sequence.
(iii) A protein encoded by a DNA consisting of a nucleotide sequence shown by nucleotide No. 1665 to 3359 in the nucleotide sequence depicted in SEQ ID No. 1, a DNA capable of hybridizing with this DNA under stringent conditions, or a DNA having at least (1) 60% identity, (2) 70% identity, (3) 80% identity, (4) 90% identity or (5) 95% identity with the nucleotide sequence.
(iv) A protein consisting of an amino acid sequence shown by amino acid No. 201 to 765 in the amino acid sequence depicted in the SEQ ID No. 2 or a protein having an amino acid sequence involving deletion, substitution or addition of one to several amino acid(s) in said amino acid sequence, which protein forms a complex with the following small subunit to show a cyclic lipopeptide acylase activity.

### Small subunit:

(i) Molecular weight: about 19 kDa (SDS-PAGE)
(ii) Amino acid analysis:
   The N-terminal amino acid sequence is Gly-Ser-Gly-Leu-Ser-Ala-Val-Ile-Arg-Tyr-Thr-Glu-Tyr-Gly-Ile-Pro-His-Ile-Val-Ala (SEQ ID No.4) or an amino acid sequence involving deletion, substitution or addition of one to several amino acid(s) in said amino acid sequence.
(iii) A protein encoded by a DNA consisting of a nucleotide sequence shown by nucleotide No. 1065 to 1664 in the nucleotide sequence depicted in SEQ ID No. 1, a DNA capable of hybridizing with this DNA under stringent conditions, or a DNA having at least (1) 60% identity, (2) 70% identity, (3) 80% identity, (4) 90% identity or (5) 95% identity with the nucleotide sequence.
(iv) A protein consisting of an amino acid sequence shown by amino acid number from -1 or 1 to 200 in the amino acid sequence depicted in SEQ ID No. 2 or a protein having an amino acid sequence involving deletion, substitution or addition of one to several amino acid(s) in said amino acid sequence, which protein forms a complex with the above-mentioned large subunit to show a cyclic lipopeptide acylase activity.

The cyclic lipopeptide acylase of the present invention is not particularly limited as to its derivation, as long as it shows the above-mentioned characteristics, and includes one derived from an organism naturally present, a natural or artificial mutant, a variant, or one derived from a transformant obtained by introducing a foreign cyclic lipopeptide acylase gene.

A method for artificially producing a mutant may be, for example, site-specific mutagenesis. More specifically, by introducing optional mutation into the nucleotide sequence depicted in SEQ ID No. 1 by this method, a mutant cyclic lipopeptide acylase can be obtained. The thus-obtained mutant cyclic lipopeptide acylase has the above-mentioned characteristics.

The cyclic lipopeptide acylase of the present invention can be obtained by suitably using a known method such as (1) a method including isolation and purification of a culture of a cell or tissue producing the enzyme as a starting material, (2) a method including chemical synthesis, (3) a method including purification from a cell processed by a genetic recombination technique and the like to express cyclic lipopeptide acylase, and the like.

For example, the cyclic lipopeptide acylase of the present invention can be isolated and purified as follows. That is, a cell expressing cyclic lipopeptide acylase is cultured in a suitable liquid medium, and extracted and purified by a known method from the obtained culture. The method for the extraction and purification is a known suitable method for the fraction containing the objective product.

The method specifically includes the following. According to a conventional method, a culture is filtrated as it is or treated by centrifugation and the like to recover a cell or a supernatant. When an enzyme is accumulated in the cell, the recovered cell is suspended in a suitable buffer and a detergent is added at a suitable concentration to solubilize the membrane. When a host cell has a cell wall, a pretreatment with a lysozyme or ultrasonication is necessary. The detergent may be, for example, sodium dodecyl sulfate (SDS), cetyltrimethylammonium bromide (CTAB) and the like, but since these have a strong protein denaturing action, a gentle nonionic detergent, such as Triton X-100 and the like, is preferably used to make a protein folded to have a biological activity. Then, the obtained crude extract solution is processed according to a suitable combination of the methods generally employed in the presence of a detergent, where necessary, to isolate and purify the enzyme.

When the enzyme is present in a culture medium, the culture is filtrated or centrifuged to remove sediment (solid such as cell and the like), leaving only the solution, which is processed according to a suitable combination of the methods generally employed to isolate and purify the objective substance. Examples of the method include a method utilizing solubility, such as salting out, solvent precipitation and the like, a method utilizing difference in molecular weights, such as dialysis, ultrafiltration, gel filtration, SDS-PAGE and the like, a method utilizing charge, such as ion exchange chromatography and the like, a method utilizing specific affinity, such as affinity chromatography and the like, a method utilizing difference in hydrophobicity, such as reverse phase high performance liquid chromatography and the like, a method utilizing difference in isoelectric points, such as isoelectric focusing method and the like, and the like. More specifically, separation and purification can be achieved by a conventional method, such as vacuum concentration, lyophilization, extraction with conventional solvent, pH adjustment, treatments using conventional adsorbent (e.g., anion exchange resin or cation exchange resin, nonionic exchange resin etc.), crystallization, recrystallization and the like. The detail is shown in the method described in WO97/32975.

The production of the cyclic lipopeptide acylase of the present invention by chemical synthesis includes using, for example, the nucleotide sequence depicted in SEQ ID No. 1, and specifying the amino. acid encoding the entirety or a part of the sequence, and synthesizing or semi-synthesizing the amino acid using a peptide synthesizer.

The cyclic lipopeptide acylase gene is generally cloned by the following method. First, the enzyme is completely or partially purified by a conventional method from a cell or tissue that produces the cyclic lipopeptide acylase and the N-terminal amino acid sequences of the large subunit and small subunit are determined by the Edman method. Each subunit is partially degraded with a sequence-specific protease and the amino acid sequence of the obtained oligopeptide is similarly determined by the Edman method. The oligonucleotide having a nucleotide sequence corresponding to the identified partial amino acid sequence is synthesized, and using this as a primer or probe, each subunit is cloned from RNA or DNA prepared from a cell or tissue producing the cyclic lipopeptide acylase by the PCR method or colony (or plaque) hybridization.

Based on the obtained nucleotide sequence of each subunit, oligonucleotide is synthesized, and using this as a primer, cyclic lipopeptide acylase is again cloned from RNA or DNA prepared from a cell or tissue producing the cyclic lipopeptide acylase by the PCR method or colony (or plaque) hybridization.

Alternatively, it is also possible to clone a DNA encoding the enzyme and/or a subunit of the enzyme by preparing an antibody against the enzyme or a subunit thereof according to a conventional method using the entirety or a part of completely or partially purified cyclic lipopeptide acylase as an antigen, and screening using the antibody from cDNA or genomic DNA library prepared from a cell or tissue producing the cyclic lipopeptide acylase.

Independently from the aforementioned method, moreover, a DNA encoding the cyclic lipopeptide acylase of the present invention can be directly cloned by the PCR method. That is, a DNA fragment containing a coding region of a cyclic lipopeptide acylase, a coding region of a large subunit or a coding region of a small subunit can be amplified by PCR according to a conventional method using a genomic DNA or CDNA (or mRNA) derived from a cell or tissue having the enzyme activity as a template and using, as a primer, a pair of suitable oligonucleotides, wherein an amplified fragment covers a coding region of a cyclic lipopeptide acylase, a coding region of a large subunit or a coding region of a small subunit.

The nucleotide sequence of the obtained DNA insert can be determined by a known sequencing technique such as Maxam-Gilbert method, dideoxy termination method and the like.

The gene encoding cyclic lipopeptide acylase of the present invention includes the entirety or a part of a DNA substantially consisting of a nucleotide sequence depicted in SEQ ID No. 1. As used herein, by the "DNA substantially consisting of" is meant a DNA having the above-mentioned specific nucleotide sequence, a DNA having a nucleotide sequence capable of hybridizing with the above-mentioned DNA having the specific nucleotide sequence under stringent conditions (in the present invention, the conditions under which a DNA having nucleotide sequence having not less than about 60% homology can hybridize, where the stringency can be adjusted by changing the temperature, salt concentration and the like during hybridization, and washing as appropriate), or a DNA having at least (1) 60% identity, (2) 70% identity, (3) 80% identity, (4) 90% identity or (5) 95% identity with the nucleotide sequence depicted in SEQ ID No. 1. The cyclic lipopeptide acylase that the gene codes for can deacylate an acyl side chain of FR901379 substance, an analog thereof and a relative thereof, such as echinocandin B, where each subunit has the aforementioned physicochemical properties.

Another embodiment of the gene encoding cyclic lipopeptide acylase of the present invention includes the entirety or a part of a DNA substantially consisting of a nucleotide sequence shown by nucleotide No. 1065 to 3359 in the nucleotide sequence depicted in SEQ ID No. 1. As used herein, the "DNA substantially consisting of" means as defined above.

Moreover, the present invention provides a gene encoding a protein of the following (a) or (b):
(a) a protein consisting of amino acid number from -1 or 1 to 765 in the amino acid sequence depicted in SEQ ID No. 2
(b) a protein having an amino acid sequence involving deletion, substitution or addition of one to several amino acid(s) in the amino acid sequence (a), which protein has a cyclic lipopeptide acylase activity.

The present invention also provides a gene encoding a large subunit and a small subunit of the above-mentioned cyclic lipopeptide acylase.

The gene of the present invention may be obtained by any method. For example, the gene includes a complementary DNA (cDNA) prepared from mRNA, a genomic DNA prepared from genomic library, a chemically synthesized DNA, a DNA obtained by amplifying by PCR using RNA or DNA as a template, a DNA constructed by a suitable combination of these methods and the like.

The present invention also relates to a recombinant vector containing any of the above-mentioned genes. The recombinant vector of the present invention is not particularly limited as long as it can be retained by replication or autonomous growth in various host cells, such as prokaryocyte and/or eucaryocyte, and encompasses a plasmid vector, a phage vector and the like. The recombinant vector can be conveniently prepared by inserting any of the above-mentioned DNAs into a cloning vector or expression vector available in this field, utilizing a suitable restriction enzyme site.

Particularly, the recombinant vector of the present invention is an expression vector functionally containing a gene encoding cyclic lipopeptide acylase. As used herein, by the "functionally" is meant that a gene (DNA) is located such that the gene is transcribed in a host cell suitable for the vector and the protein encoded by the gene can be produced. Preferably, it is a vector containing an expression cassette wherein a promoter region, an initiation codon and a gene encoding cyclic lipopeptide acylase or each subunit thereof, a termination codon and a terminator region are sequentially arranged. The expression vector to be used is not particularly limited as long as it contains a promoter region that functions in various host cells of prokaryocyte and/or eucaryocyte and expresses the gene downstream thereof and a signal that terminates the transcription of the gene, namely, a terminator region, and the promoter region and the terminator region are connected via a sequence containing at least one unique restriction enzyme recognition site. It is preferable that it further contain a selection marker gene for selection of a transformant. Where desired, the expression vector may contain an initiation codon and a termination codon downstream of the promoter region and upstream of the terminator region, respectively.

When an expression vector containing a gene encoding a large subunit (or small subunit) of cyclic lipopeptide acylase is used for the production of cyclic lipopeptide acylase, an expression vector containing, in addition to the DNA, a DNA encoding a small subunit (or large subunit) in a state that affords expression in a host cell is used. The DNA encoding a large subunit and a DNA encoding a small subunit may be placed under the control of different promoters or placed in tandem under the control of the same promoter.

For secretion of cyclic lipopeptide acylase into a medium to obtain the acylase, the expression vector of the present invention preferably contains functionally the signal sequence. The signal sequence is not particularly limited as long as the protein secretion mechanism of the host cell can be recognized. When the host cell is *Actinomycetes,* those belonging to the genus *Streptomyces* are preferable, which are derived from the same origin as the gene encoding cyclic lipopeptide acylase of the present invention. The signal sequence is cleaved and removed by protease in the cell and mature protein is secreted out of the cell.

When bacteria are used as a host cell, an expression vector generally needs to contain, in addition to the above-mentioned promoter region and terminator region, a replicatable unit that can autonomously replicate in a host cell. A promoter region contains a promoter, an operator and a Shine-Dalgarno (SD) sequence. For example, when the host is *E. coli,* Trp promoter, lac promoter, recA promoter, lpp promoter, tac promoter and the like are used as the promoter region, when the host is *Bacillus subtilis,* SPO1 promoter, SPO2 promoter, penP promoter and the like are used as the promoter region and when the host is *Actinomycetes,* melC, tipA, ermE, aphI and the like are used. As the terminator region, conventionally used natural or synthetic terminators can be used. As the selection marker gene, genes resistant to various drugs such as tetracycline, ampicillin, kanamycin, thiostrepton and the like can be used. As the initiation codon, ATG is generally used, but GTG may be also used. As the termination codon, conventional TGA, TAA and TAG can be used.

When the gene encoding cyclic lipopeptide acylase of the present invention is prepared from a genomic DNA derived from a cell or tissue producing the enzyme and is obtained in the form containing the original promoter and terminator regions, the expression vector of the present invention can be prepared by inserting the DNA into a suitable site of a known cloning vector capable of replicatable retention or autonomous growth in a host cell to be transformed. The cloning vector to be used when the host is bacteria includes pBR vector, pUC vector and the like derived from *E. coli,* pUB110, pTP5, pC194 and the like derived from *Bacillus subtilis,* pIJ702, pSK1, pSK2, SCP2, SCP1.2, pGA482, pMCXpress and the like derived from *Actinomycetes.*

The transformant of the present invention can be prepared by transforming a host cell with a recombinant vector containing a gene encoding cyclic lipopeptide acylase of the present invention. The host cell is not particularly limited as long as it is compatible with the recombinant vector to be used and can be transformed. Various cells generally used in this field, such as naturally occurring cell, artificially prepared mutant cell, recombinant cell and the like can be used. It is preferably bacteria, particularly *E. coli, Bacillus subtilis* and *Actinomycetes* and the like, and more preferably bacteria belonging to the genus *Streptomyces,* which is one kind of *Actinomycetes.*

A recombinant vector can be introduced into a host cell by a conventionally known method. For example, when the host cell is *E. coli*, *Bacillus subtilis* and the like, the method of Cohen et al. [Proc. Natl. Acad. Sci. USA, 69: 2110 (1972)], protoplast method [Mol. Gen. Genet., 168: 111 (1979)], competent method [J. Mol. Biol., 56: 209 (1971)] and the like can be used.

When the host is *Actinomycetes,* particularly bacteria belonging to the genus *Streptomyces,* a method (PEG-assisted protoplast transformation) described in "Transformation Genetic Manipulation of Streptomyces. A Laboratory Manual. The John Innes Foundation, Norwich, UK, 1985" and the like can be used.

The present invention also provides cyclic lipopeptide acylase.

The cyclic lipopeptide acylase of the present invention can be produced by culturing any of the transformants containing an expression vector functionally containing the above-mentioned gene encoding cyclic lipopeptide acylase in a suitable medium and harvesting the enzyme from the obtained culture. The isolation and purification can be performed by suitably combining various separation techniques generally used as mentioned above according to the fraction showing a cyclic lipopeptide acylase activity. When produced by each subunit unit, an expression vector functionally containing the gene encoding each subunit is used for similar production.

The nutrient medium to be used contains, as sources of carbon, carbohydrates such as glucose, xylose, galactose, glycerine, starch, dextrin and the like, and as other sources of carbon, maltose, rhamnose, raffinose, arabinose, mannose, salicin, sodium succinate, fructose, mannitol, glucitol, lactose, sorbose, sucrose and the like can be used.

The preferred sources of nitrogen may include inorganic and organic nitrogen sources such as ammonium sulfate, ammonium chloride, casein hydrolysate, yeast extract, polypeptone, bactotrypton, beef extract, soybean powder, wheat germ, potetoprotein, rice bran, peanut powder, gluten, corn extract and the like. Where desired, other nutrient sources [e.g., inorganic salt (e.g., sodium diphosphate, potassium diphosphate, potassium phosphate, magnesium chloride, magnesium sulfate, calcium chloride), vitamins (e.g., vitamin B1), antibiotics (e.g., ampicillin, kanamycin, thiostrepton) and the like] may be added to a medium.

If necessary, especially when the culture medium foams seriously a defoaming agent, such as liquid paraffin, fatty oil, plant oil or silicone, or the like may be added.

A transformant can be cultured generally at pH 4 - 9, preferably 6 - 7, at 15 - 35°C, preferably 25 - 35°C for 10 - 144 hours.

According to the method for deacylating a side chain acyl group of a cyclic lipopeptide substance of the present invention, any of the above-mentioned transformants containing an expression vector functionally containing a DNA encoding cyclic lipopeptide acylase is cultured in a suitable medium, and using the obtained culture as it is, cyclic lipopeptide substance is brought into contact therewith to deacylate a side chain acyl group of the substance into an amino group. Alternatively, when the enzyme activity is found in the intracellular fraction of the transformant, by bringing cyclic lipopeptide substance into contact with the cell extract to deacylate a side chain acyl group of the substance into an amino group.

When the cyclic lipopeptide substance is brought into contact with cell extract, after completion of the culture, the culture liquid is centrifuged or filtrated to recover the cells, which are suspended in a suitable buffer, such as acetate buffer. By ultrasonication and the like, the cell are ruptured and centrifuged, and the obtained supernatant is used as a cell extract.

A deacylated cyclic lipopeptide substance can be also obtained by culturing a host cell producing a cyclic lipopeptide acylase in the presence of a cyclic lipopeptide substance.

The cyclic lipopeptide substance to be the substrate of the cyclic lipopeptide acylase of the present invention has a polypeptide ring and an "acylamino group" on the ring as a side chain. This substance may further have a different side chain. For example, the substance disclosed in WO97/32975 is mentioned.

The FR901379 substance which is one example of the "cyclic lipopeptide substance" is a known substance (JP-A-3-184921) having an antifungal activity, which is produced by a *microorganism Coleophoma* sp. F-11899 strain (FERM BP-2635; deposited at the Fermentation Research Institute, Agency of Industrial Science and Technology, the Ministry of International Trade and Industry, 1-3, Higashi 1-chome, Tsukuba-shi, Ibaraki-ken, Japan, date of original deposit:
October 26, 1988), and is a compound represented by the following structural formula [Ia]:

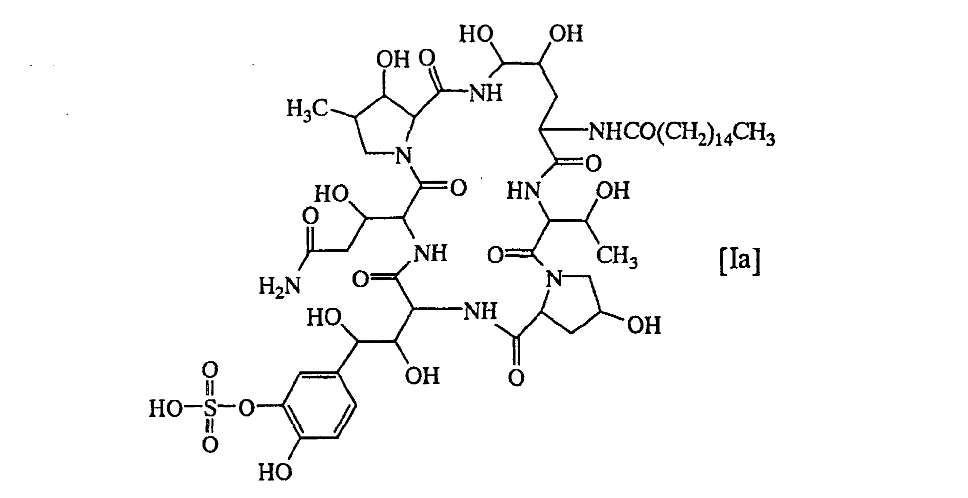

The FR901379 substance analog refers to a compound of the following formula [I] or a salt thereof. 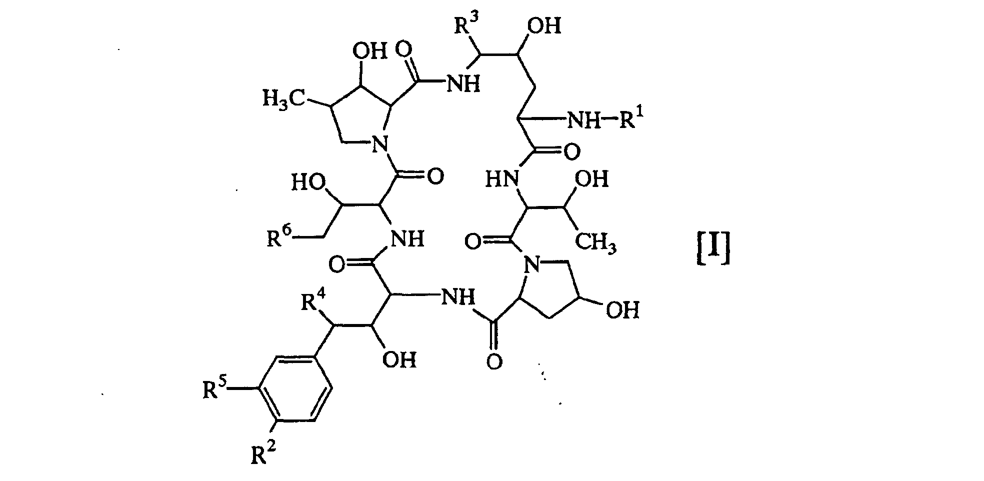 wherein R¹ is acyl group, R² is hydroxyl group or acyloxy group, R³ is hydrogen or hydroxyl group, R⁴ is hydrogen or hydroxyl group, R⁵ is hydrogen or hydroxysulfonyloxy group and R⁶ is hydrogen or carbamoyl group.

The cyclic lipopeptide acylase of the present invention deacylates a side chain "acylamino group" of a cyclic lipopeptide substance to lead to an "amino group".
Specifically, it is an acylase that deacylates a palmitoyl side chain of FR901379 substance or a salt thereof, or an acyl side chain of an analog of acylase FR901379 substance analog of the aforementioned formula [I], inclusive of FR901379 substance, or a salt thereof to produce a cyclic lipopeptide substance, specifically a compound (FR179642 substance) of the following structural formula [IIa] or a salt thereof 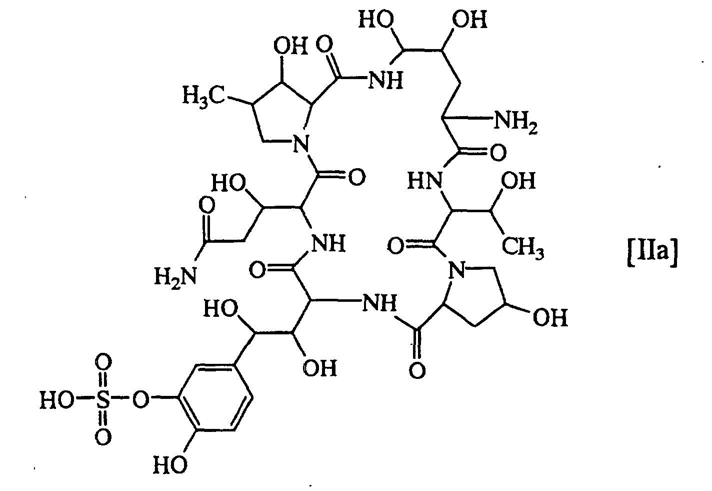 or an FR179642 analog of the following formula [II], inclusive of an FR179642 substance, or a salt thereof 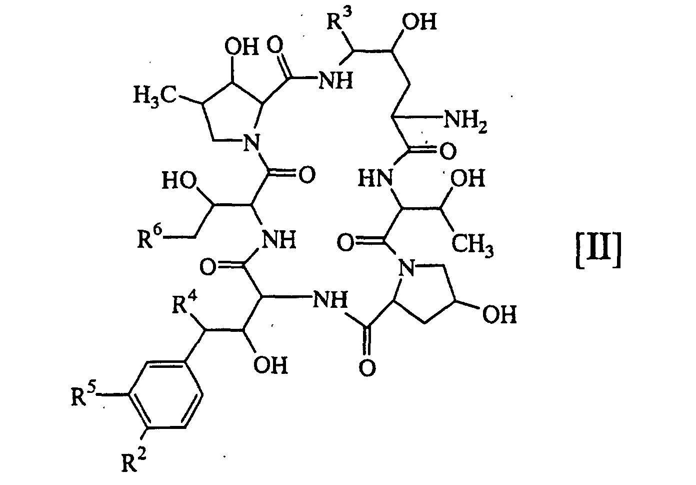 wherein R², R³, R⁴, R⁵ and R⁶ mean the same groups as defined above.

Preferable salts of the compound [I] and [II] are conventional non-toxic mono or di salts, which are exemplified by metal salt, such as alkali metal salt (e.g., sodium salt, potassium salt etc.), alkaline earth metal salt (e.g., calcium salt, magnesium salt etc.), ammonium salt, salts with organic base (e.g., trimethylamine salt, triethylamine salt, pyridine salt, picoline salt, dicyclohexylamine salt, N,N'-dibenzylethylenediamine salt etc.) and the like, organic acid addition salt (e.g., formate, acetate, trifluoroacetate, maleate, tartrate, methanesulfonate, benzenesulfonate, toluenesulfonate etc.), inorganic acid addition salt (e.g., hydrochloride, hydrobromate, hydroiodide, sulfate, phosphate etc.), salts with amino acid (e.g., arginine, asparatic acid, glutamic acid etc.), and the like.

After the completion of the deacylation, the deacylated cyclic lipopeptide substance, specifically, an FR179642 analog (inclusive of FR179642 substance) of the formula [II] can be separated and purified from the reaction mixture by a conventionally known separation and purification methods, such as vacuum concentration, lyophilization, extraction, pH adjustment, absorption resin, ion exchange resin, crystallization, recrystallization and the like, which are combined as appropriate.

### Examples

The present invention is explained in detail in the following by way of Examples. It is needless to say that the present invention is not limited by these Examples.

Many techniques, reactions and analysis methods used in the present invention are known *per se* to those of ordinary skill in the art. Unless particularly specified, the enzymes, plasmids, hosts and the like are commercially available.

### Example 1

Cloning of cyclic lipopeptide acylase (FR901379 acylase) produced by *Streptomyces sp.* No.6907 strain

### (1) Preparation of chromosomal DNA of Streptomyces sp. No.6907 strain

A spoonful of a cryopreserved solution of *Streptomyces sp.* No. 6907 strain (FERM BP-5809; WO97/32975; deposited at the Fermentation Research Institute, Agency of Industrial Science and Technology, the Ministry of International Trade and Industry, 1-3, Higashi 1-chome, Tsukuba-shi, Ibaraki-ken, Japan, date of original deposit March 8, 1996, date of international deposit (transfer) February 3, 1997) was cultured in a medium (pH 6.5) containing 0.3% yeast extract, 0.5% peptone, 0.3% malt infusion, 1% glucose, 5% sucrose, 5 mM MgCl₂ and 0.5% glycine at 30°C for 48 h. The culture solution (10 ml) was centrifuged (5,000 rpm, 10 min) to collect the cells. The cells were treated using QIAGEN Genomic tip 20/G (QIAGEN Inc.) according to the protocol to give chromosomal DNA.

### (2) Analysis of small subunit

### (i) Design of primer for PCR from known amino terminal amino acid sequence of small subunit

The following forward primer (SF3) and reverse primer (SR2) were designed from the amino terminal amino acid sequence of FR901379 acylase small subunit
Gly Ser Gly Leu Ser Ala Val Ile Arg Tyr Thr Glu Tyr Gly Ile Pro His His Val Ala (SEQ ID No. 5) (WO97/32975 publication) [Note that the amino terminal amino acid sequence of this small subunit was analyzed in detail later and the accurate sequence was found to be Gly Ser Gly Leu Ser Ala Val Ile Arg Tyr Thr Glu Tyr Gly Ile Pro His Ile Val Ala (SEQ ID No. 4).]. SF3 -CTS TCS GCS GTS ATC (SEQ ID No. 6)
SR2 -GTG GTG SGG GAT SCC (SEQ ID No. 7)
S: C or G

### (ii) PCR using a primer designed from known amino terminal amino acid sequence of small subunit

PCR was performed using chromosomal DNA (100 ng) of *Streptomyces* sp. No.6907 strain prepared in the above-mentioned (1) and each primer (1 nmol) designed in the above-mentioned (i) in GeneAmp PCR System Model 2400 (Perkin-Elmer, Inc.). The reaction mixture (50 µl [in PCR buffer, 0.2 mM each dNTPs and KOD Dash (Toyobo Co., Ltd.), 1.5 unit] was subjected to 30 cycles of PCR consisting of 20 seconds of denaturing at 98°C, 2 seconds of annealing at 60°C, and 10 seconds of polymerization at 74°C. After amplification, a fragment (45 bp) encoding a part of the known amino terminal of a small subunit was isolated by 5% agarose gel electrophoresis.

### (iii) Cloning of PCR fragment

The PCR amplified fragment (45 bp) isolated in the above-mentioned (ii) was treated using a pCR-Script Amp Cloning Kit (Stratagene) according to the protocol to give plasmid p3S4, in which the fragment was inserted into pCR-Script Amp SK(+).

### (iv) Analysis of nucleotide sequence

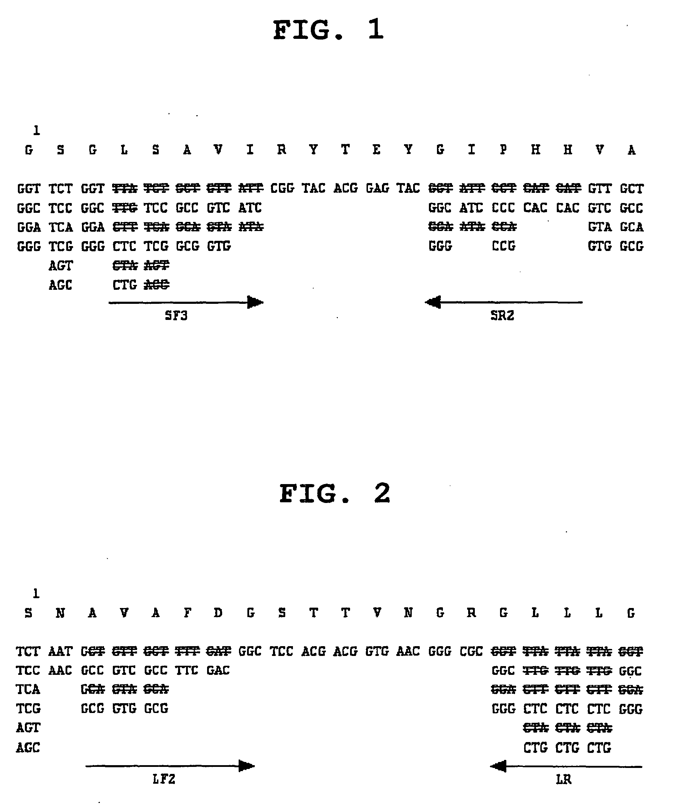

The nucleotide sequence of plasmid p3S4 obtained in the above-mentioned (iii) was analyzed with a 310 DNA sequencer (Perkin-Elmer, Inc.) using a reverse primer (New England Biolabs Inc.), which is M13 sequencing primer, according to the dideoxy termination method according to the attached protocol for sequencing. The results are shown in Fig. 1.

### (3) Analysis of large subunit

### (i) Design of primer for PCR from known amino terminal amino acid sequence of large subunit

The following forward primer (LF2) and reverse primer (LR) were designed from the amino terminal amino acid sequence of FR901379 acylase large subunit
Ser Asn Ala Val Ala Phe Asp Gly Ser Thr Thr Val Asn Gly Arg Gly Leu Leu Leu Gly (SEQ ID No. 3) (WO97/32975 publication) LF2 - CS GTS GCS TTC GAC GG (SEQ ID No. 8)
LR - SCC SAG SAG SAG SCC (SEQ ID No. 9)
S: C or G.

### (ii) PCR using a primer designed from known amino terminal amino acid sequence of large subunit

PCR was performed using chromosomal DNA (100 ng) of *Streptomyces sp.* No.6907 strain prepared in the above-mentioned (1) and each primer (1 nmol) designed in the above-mentioned (3)(i) in GeneAmp PCR System Model 2400 (Perkin-Elmer, Inc.). The reaction mixture (50 µl [in PCR buffer, 0.2 mM each dNTPs and KOD Dash (Toyobo Co., Ltd.), 1.5 unit] was subjected to 30 cycles of PCR consisting of 20 seconds of denaturing at 98°C, 2 seconds of annealing at 65°C, and 10 seconds of polymerization at 74°C. After amplification, a fragment (53 bp) encoding a part of the known amino terminal of a large subunit was isolated by 5% agarose gel electrophoresis.

### (iii) Cloning of PCR fragment

The PCR amplified fragment (53 bp) isolated in the above-mentioned (3)(ii) was treated using a pCR-Script Amp Cloning Kit (Stratagene) according to the protocol to give plasmid p3L1 in which the fragment was inserted into pCR-Script Amp SK(+).

### (iv) Analysis of nucleotide sequence

The nucleotide sequence of plasmid p3L1 was analyzed with a 310 DNA sequencer (Perkin-Elmer, Inc.) using a reverse primer (New England Biolabs), which is M13 sequencing primer, according to the dideoxy termination method according to the attached protocol for sequencing. The results are shown in Fig. 2.

### (4) Analysis of FR901379 acylase

### (i) Design of primer for PCR between small and large subunits

In the acylases heretofore known, most of them are encoded in the order of a small subunit and a large subunit. Therefore, a forward primer 20S was designed based on the nucleotide sequence determined by PCR in the known amino acid sequence of the small subunit. Similarly, a reverse primer 19L was designed based on the nucleotide sequence determined by PCR in the known amino acid sequence of the large subunit. 20S -ATC CGG TAC ACG GAG TAC GG (SEQ ID No. 10)
19L -C GTT CAC CGT CGT GGA GCC (SEQ ID No. 11)

### (ii) PCR between small and large subunits

PCR was performed using chromosomal DNA (100 ng) of *Streptomyces sp.* No. 6907 strain prepared in the above-mentioned (1) and each primer (20 pmol) designed in the above-mentioned (4)(i) in GeneAmp PCR System Model 2400 (Perkin-Elmer, Inc.). The reaction mixture (50 µl [in PCR buffer, 0.2 mM each dNTPs and KOD Dash (Toyobo Co., Ltd.), 2.5 units] was subjected to 30 cycles of PCR consisting of 20 seconds of denaturing at 98°C, 2 seconds of annealing at 70°C, and 20 seconds of polymerization at 74°C. After amplification, a fragment (about 600 bp) encoding a part of the acylase was isolated by 2% agarose gel electrophoresis.

### (iii) Cloning of PCR fragment

The PCR amplified fragment (about 600 bp) isolated in the above-mentioned (4)(ii) was treated using a pCR-Script Amp Cloning Kit (Stratagene) according to the protocol to give plasmid pSL1 in which the fragment was inserted into pCR-Script Amp SK(+).

### (iv) Analysis of nucleotide sequence

The nucleotide sequence of plasmid pSL1 obtained in the above-mentioned (4)(iii) was analyzed with a 310 DNA sequencer (Perkin-Elmer, Inc.) using a forward primer and a reverse primer (New England Biolabs Inc.), which are M13 sequencing primers, according to the dideoxy termination method according to the attached protocol for sequencing. As a result, a part of the gene seemingly the acylase was obtained.

### (5) Preparation of chromosomal DNA library

The chromosomal DNA (1 µg) of *Streptomyces sp.* No. 6907 strain prepared in the above-mentioned (1) was treated with Sau3A I (100 mU) at 37°C for 10 min for partial digestion. The cosmid pcos6EMBL (1 µg, see Gene, 57, 229-237 (1987)) was treated with BamH I 5U at 37°C for 1 h. The both treated solutions were subjected to ethanol precipitation and dissolved in 2-fold diluted TE (5 µl, 5 mM Tris-HCl (pH 8.0), 0.5 mM), to which 10 × T4 DNA ligase buffer (0.7 µl, 660 mM Tris-HCl (pH 7.6), 66 mM MgCl₂, 100 mM DTT, 1 mM ATP) and T4 DNA ligase (0.7 µl) were added. The mixture was incubated at 22°C for 3 h. This ligation solution (3 µl) was subjected to in vitro packaging using GIGAPACK III XL Packaging Extract (Stratagene) according to the protocol. This packaging solution was brought into contact with an indicator strain *E. coli* XL-1 Blue MRA to construct the cosmid library.

### (6) Screening by colony direct PCR

The cosmid clones (480 clones) obtained above were subjected to colony direct PCR using primer 20S and 19L (each 20 pmol) and GeneAmp PCR System Model 2400 (Perkin-Elmer, Inc.). The reaction mixture (20 µl [in PCR buffer, 0.2 mM each dNTPs and KOD Dash (Toyobo Co., Ltd.), 2.5 units] was subjected to 30 cycles of PCR consisting of 20 seconds of denaturing at 98°C, 2 seconds of annealing at 68°C, and 20 seconds of polymerization at 74°C. As a result, a cosmid clone No. 133 wherein about 600 bp fragment was specifically amplified was obtained.

### (7) Subcloning of cosmid clone No. 133

The cosmid clone No. 133 was digested with EcoR I and Pst I and the obtained about 8 kb fragment was inserted into the EcoR I/Pst I site of pUC18 to give plasmid pEP1. The plasmid pEP1 was digested with EcoR I and BamH I and the obtained about 5.5 kb fragment was inserted into the EcoR I/BamH I site of pUC18 to give plasmid pEB.

### (8) Nucleotide sequence analysis

The nucleotide sequence of plasmid pEB was analyzed with a 310 DNA sequencer (Perkin-Elmer, Inc.) using a forward primer and a reverse primer (New England Biolabs Inc.), which are M13 sequencing primers, and synthetic oligonucleotide shown in Table 1, according to the dideoxy termination method according to the attached protocol for sequencing.

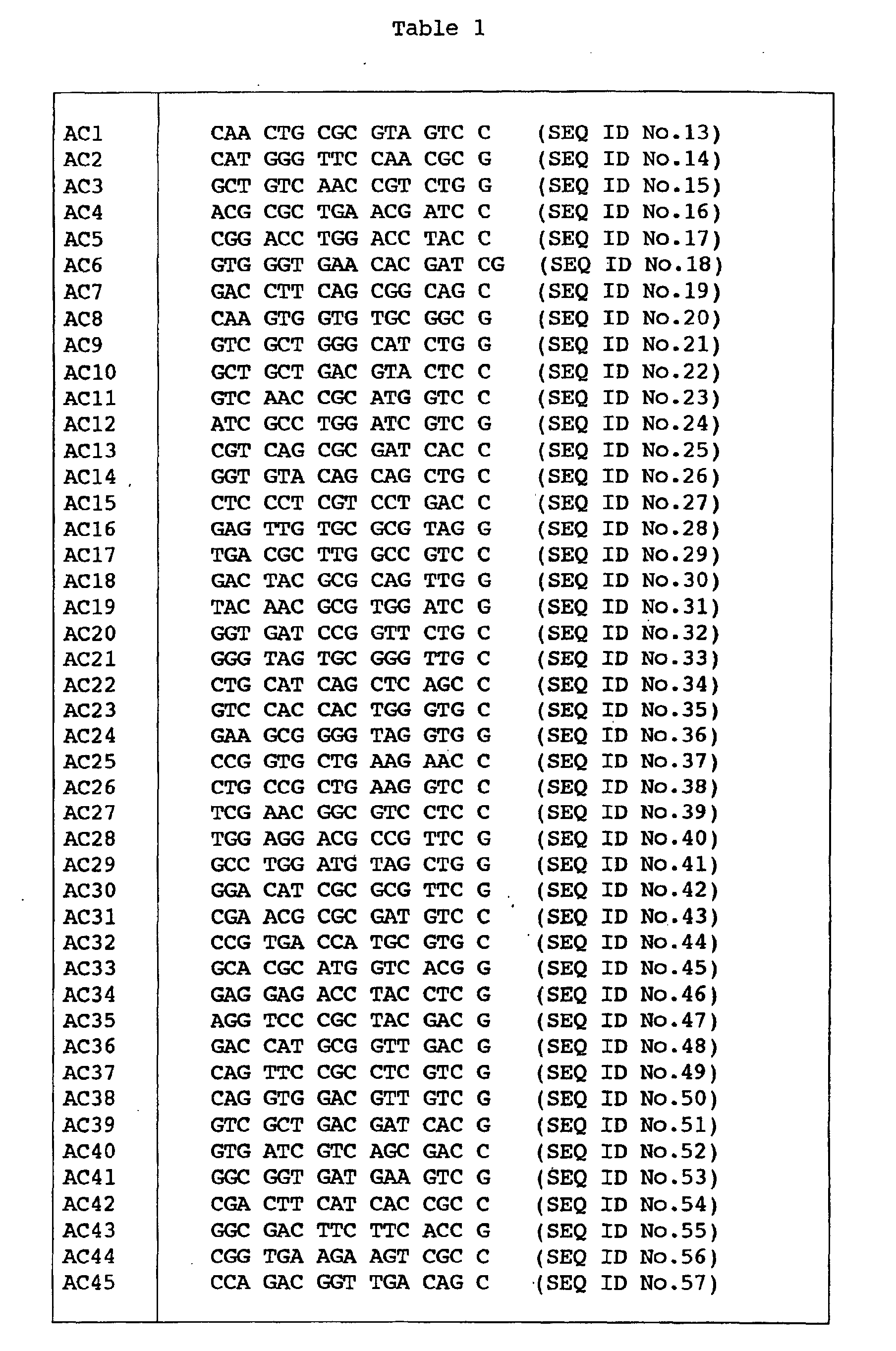

From the analysis of the nucleotide sequence, ORF was found, in which DNA sequences corresponding to the amino terminal amino acid sequences of a small subunit and a large subunit were included entirely. The nucleotide sequence of plasmid pEB containing the acylase gene is shown in SEQ ID No. 1.

### Example 2 Expression of cyclic lipopeptide acylase in a host cell

### (1) Construction of Streptomyces lividans 1326/pIJ702-SB

Plasmid pSB wherein EcoR I site of plasmid pEB was changed to Sac I site was to-be constructed. First, a synthetic oligomer (100 pmol, AAT TGA GCT C; SEQ ID No. 12) was treated with 30U T4 polynucleotide kinase at 37°C for 1 h to phosphorylate the 5'-OH terminal. The reaction mixture was heated at 70°C for 10 min to inactivate the T4 polynucleotide kinase. Separately, 1 µg of plasmid pEB was treated with 5U EcoR I and with 1U Bacterial Alkaline Phosphatase (BAP) at 37°C for 1 h to dephosphorylate the 5' terminal. These two treatment solutions were ligated with Ligation High (Toyobo Co., Ltd.) to construct plasmid pSB.

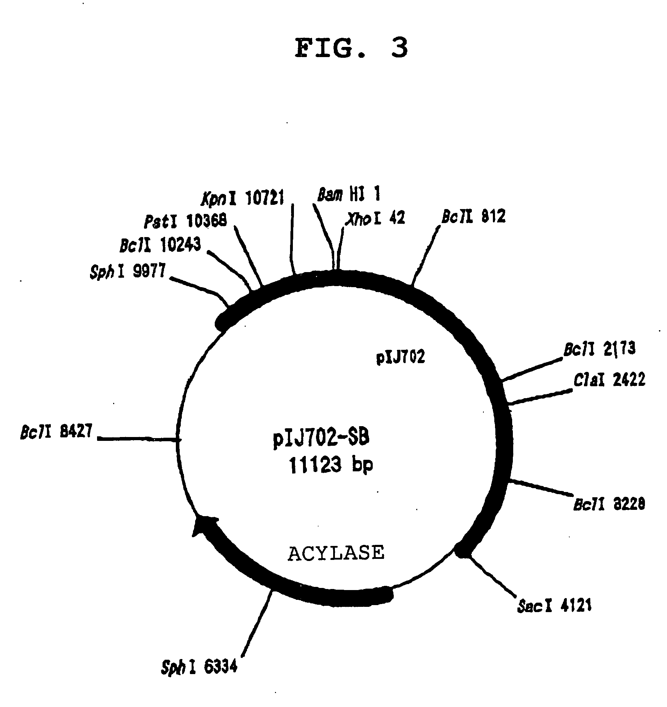

The plasmid pSB (5 µg) was treated with 20 U Sac I and 20U BamH I to give a 5.7 kb Sac I-BamH I fragment containing an acylase gene. pIJ702 (2 µg)(ATCC 35287), which is a vector for *Actinomycetes,* was treated with 10 U Sac I and 10 U Bgl II and ligated with 5.7 kb Sac I-BamH I fragment containing an acylase gene prepared beforehand in the presence of Ligation High (Toyobo Co., Ltd.). The ligation solution was used to transform *Streptomyces lividans* 1326 strain (J. General Microbiology 1983, 129, 2703-2713) according to the method described in "Genetic Manipulation of Streptomyces. A Laboratory Manual. The John Innes Foundation, Norwich, UK, 1985". Of the obtained transformed strains, one strain was taken as *Streptomyces lividans* 1326/pIJ702-SB (Fig. 3).

### (2) Culture of the transformant and expression of FR901379 acylase

A medium (10 ml) containing 5% sucrose, 1% glucose, 0.3% yeast extract (Difco), 0.5% bactopeptone (Difco), 0.3% meat extract (Difco), 5 mM MgCl₂, 0.5% glycine and 50 µg/ml of thiostrepton (pH 6.5) was placed in a 100 ml Erlenmeyer flask, in which the bacterial cells (5 mm square) of the transformed strain *Streptomyces lividans* 1326/pIJ702-SB were inoculated. The cells were cultured at 30°C for 3 days (260 rpm) and the FR901379 acylase activity of the culture solution was determined. As a result, an activity to produce 30 mg of FR179642 (deacylated FR901379 substance) per 1 ml of the culture solution per 1 h was found.

The culture time until the acylase activity reached the maximum was shortened to 2 - 3 days for transformed strain *Streptomyces lividans* 1326/pIJ702-SB. Since it was 7 days for *Streptomyces sp.* No. 6907, the time was shorter than half.

The acylase activity was determined as follows.

### Measurement of acylase activity>

The culture solution (0.1 ml) was added to a solution consisting of 100 mg/ml aqueous FR901379 (see WO97/32975) solution (0.1 ml), phosphate buffer (pH 6.0, 0.1 ml), methanol (0.1 ml) and distilled water (0.6 ml) and reacted at 37°C (125 rpm). After 15 min, 4% acetic acid (1 ml) and distilled water (2 ml) were added to terminate the reaction. The produced FR179642 (deacylated FR901379 substance) was quantitatively determined by high performance liquid chromatography (HPLC) under the following conditions.
column; Kaseisorb LC PO Super (4.6 mm I.D. x 250 mm) (Tokyo Kasei Kogyo Co., Ltd)
column temperature; 50°C
eluate; distilled water:methanol:phosphoric acid = 960:40:1 flow rate; 1 ml/min
detection; UV-215 nm

### Example 3

### (1) Preparation of fermented solution

The PM-1 medium (50 mL) (6% *Nisshoku* #3600, 3% defatted soybean meal powder, 0.5% CaCO₃, 0.005% thiostrepton, pH non-adjusted) containing thiostrepton (50 µg/mL) was inoculated in a 500 mL flask, and after inoculating bacterial cells (5 mm square) of the transformed strain *Streptomyces lividans* 1326/pIJ702-SB, the cells were cultured at 30°C for 3 days. The culture (2.5 mL) was inoculated in a 500 mL flask containing 50 mL of SG medium (8% maltose, 3% defatted soybean meal powder, 3% defatted wheat germ, 0.5% CaCO₃, 0.005% thiostrepton, pH non-adjusted) and cultured at 30°C for 3 days.

### (2) Purification of FR901379 acylase

4M KCl (8 mL) was added to the fermented solution (24 mL) and the mixture was left standing overnight at 4°C, which was followed by centrifugation (10,000 rpm, 10 min) to give a supernatant and used as a KCl extract solution. This KCl extract solution was concentrated 10-fold with Microcon 50 (Millipore Corporation) and returned to the original liquid amount with 0.5M sodium phosphate buffer (pH 6.0). This step was repeated twice to remove low molecular weight protein.

### (3) SDS-PAGE analysis

SDS-PAGE analysis was performed using multigel 10/20 or 15/25 (Daiichi Pure Chemicals Co., Ltd.; acrylamide 10-20% or 15-25% gradient gel).

### (4) Measurement of FR901379 acylase activity

Measured according to Example 2.

### (5) Quantification of protein

Measured by DC Protein Assay (Lowry method; Biolad) using albumin as a standard.

### (6) Amino terminal amino acid sequence analysis

The protein in a gel after SDS-PAGE was transferred to PVDF membrane like electrophoresis by Horizblot (Atto Co. Ltd.) and stained with CBB. The objective band was cleaved with scissors and subjected to amino terminal amino acid sequence analysis.

From the analysis results, it was found that 45% of the cyclic lipopeptide acylase small subunit had an N-terminal amino acid sequence described in SEQ ID No. 4 and 55% thereof had an N-terminal amino acid sequence wherein a serine residue was added to the N terminal thereof.

### Industrial Applicability

The recombinant cyclic lipopeptide acylase of the present invention, which is obtained from a transformant obtained by introducing a gene encoding cyclic lipopeptide acylase, has the same level of activity as does an acylase obtained by culturing a natural separation strain that produces cyclic lipopeptide acylase such as conventional *Streptomyces sp.* No. 6907 strain, *Streptomyces anulatus* No. 8703 strain and the like, and the use of the transformant has made it possible to shorten the time necessary for culture (time necessary for producing cyclic lipopeptide acylase).

This application is based on patent application No. 189644/1999 filed in Japan, the contents of which are hereby incorporated by reference.

### Sequence Listing Free Text

SEQ ID NO: 6 Oligonucleotide designed to act as PCR primer (forward) to amplify the DNA coding N-terminal amino acid sequences of FR901379 acyrase small subunit.

SEQ ID NO: 7 Oligonucleotide designed to act as PCR primer (reverse) to amplify the DNA coding N-terminal amino acid sequences of FR901379 acyrase small subunit.

SEQ ID NO: 8 Oligonucleotide designed to act as PCR primer (forward) to amplify the DNA coding N-terminal amino acid sequences of FR901379 acyrase large subunit.

SEQ ID NO: 9 Oligonucleotide designed to act as PCR primer (reverse) to amplify the DNA coding N-terminal amino acid sequences of FR901379 acyrase large subunit.

SEQ ID NO: 10 Oligonucleotide designed to act as PCR primer (forward) to amplify the DNA coding amino acids between FR901379 acyrase small subunit and large subunit.

SEQ ID NO: 11 Oligonucleotide designed to act as PCR primer (reverse) to amplify the DNA coding amino acids between FR901379 acyrase small subunit and large subunit.

SEQ ID NO: 12 Oligonucleotide designed for use in changing restriction site from EcoR I site to Sac I site.

SEQ ID NO: 13 Oligonucleotide designed to act as sequencing primer.

SEQ ID NO: 14 Oligonucleotide designed to act as sequencing primer.

SEQ ID NO: 15 Oligonucleotide designed to act as sequencing primer.

SEQ ID NO: 16 Oligonucleotide designed to act as sequencing primer.

SEQ ID NO: 17 Oligonucleotide designed to act as sequencing primer.

SEQ ID NO: 18 Oligonucleotide designed to act as sequencing primer.

SEQ ID NO: 19 Oligonucleotide designed to act as sequencing primer.

SEQ ID NO: 20 Oligonucleotide designed to act as sequencing primer.

SEQ ID NO: 21 Oligonucleotide designed to act as sequencing primer.

SEQ ID NO: 22 Oligonucleotide designed to act as sequencing primer.

SEQ ID NO: 23 Oligonucleotide designed to act as sequencing primer.

SEQ ID NO: 24 Oligonucleotide designed to act as sequencing primer.

SEQ ID NO: 25 Oligonucleotide designed to act as sequencing primer.

SEQ ID NO: 26 Oligonucleotide designed to act as sequencing primer.

SEQ ID NO: 27 Oligonucleotide designed to act as sequencing primer.

SEQ ID NO: 28 Oligonucleotide designed to act as sequencing primer.

SEQ ID NO: 29 Oligonucleotide designed to act as sequencing primer.

SEQ ID NO: 30 Oligonucleotide designed to act as sequencing primer.

SEQ ID NO: 31 Oligonucleotide designed to act as sequencing primer.

SEQ ID NO: 32 Oligonucleotide designed to act as sequencing primer.

SEQ ID NO: 33 Oligonucleotide designed to act as sequencing primer.

SEQ ID NO: 34 Oligonucleotide designed to act as sequencing primer.

SEQ ID NO: 35 Oligonucleotide designed to act as sequencing primer.

SEQ ID NO: 36 Oligonucleotide designed to act as sequencing primer.

SEQ ID NO: 37 Oligonucleotide designed to act as sequencing primer.

SEQ ID NO: 38 Oligonucleotide designed to act as sequencing primer.

SEQ ID NO: 39 Oligonucleotide designed to act as sequencing primer.

SEQ ID NO: 40 Oligonucleotide designed to act as sequencing primer.

SEQ ID NO: 41 Oligonucleotide designed to act as sequencing primer.

SEQ ID NO: 42 Oligonucleotide designed to act as sequencing primer.

SEQ ID NO: 43 Oligonucleotide designed to act as sequencing primer.

SEQ ID NO: 44 Oligonucleotide designed to act as sequencing primer.

SEQ ID NO: 45 Oligonucleotide designed to act as sequencing primer.

SEQ ID NO: 46 Oligonucleotide designed to act as sequencing primer.

SEQ ID NO: 47 Oligonucleotide designed to act as sequencing primer.

SEQ ID NO: 48 Oligonucleotide designed to act as sequencing primer.

SEQ ID NO: 49 Oligonucleotide designed to act as sequencing primer.

SEQ ID NO: 50 Oligonucleotide designed to act as sequencing primer.

SEQ ID NO: 51 Oligonucleotide designed to act as sequencing primer.

SEQ ID NO: 52 Oligonucleotide designed to act as sequencing primer.

SEQ ID NO: 53 Oligonucleotide designed to act as sequencing primer.

SEQ ID NO: 54 Oligonucleotide designed to act as sequencing primer.

SEQ ID NO: 55 Oligonucleotide designed to act as sequencing primer.

SEQ ID NO: 56 Oligonucleotide designed to act as sequencing primer.

SEQ ID NO: 57 Oligonucleotide designed to act as sequencing primer.

## Claims

1. A gene encoding cyclic lipopeptide acylase, which comprises the entirety or a part of the following (a), (b) or (c):
(a) a DNA consisting of the nucleotide sequence depicted in SEQ ID No. 1
(b) a DNA capable of hybridizing with the DNA of the above-mentioned (a) under stringent conditions
(c) a DNA having at least (1) 60% identity, (2) 70% identity, (3) 80% identity, (4) 90% identity or (5) 95% identity with the nucleotide sequence depicted in SEQ ID No. 1.

2. A gene encoding a protein of the following (a) or (b) or a part thereof:
(a) a protein consisting of the amino acid sequence depicted in SEQ ID No. 2
(b) a protein having an amino acid sequence involving deletion, substitution or addition of one to several amino acid(s) in the amino acid sequence (a), which protein has a cyclic lipopeptide acylase activity.

3. A recombinant vector comprising the gene of claim 1 or 2.

4. An expression vector functionally comprising the gene of claim 1 or 2.

5. A transformant obtained by transforming a host cell with the vector of claim 3 or 4.

6. A method of producing cyclic lipopeptide acylase, which comprises
culturing a host cell transformed with the expression vector of claim 4, and
harvesting, from the obtained culture, cyclic lipopeptide acylase capable of catalyzing a reaction to deacylate a side chain acylamino group of a cyclic lipopeptide substance into an amino group.

7. A cyclic lipopeptide acylase produced by the production method of claim 6.

8. A gene encoding cyclic lipopeptide acylase, which comprises the entirety or a part of the following (a), (b) or (c):
(a) a DNA consisting of a nucleotide sequence shown by nucleotide No. 1065 to 3359 in the nucleotide sequence depicted in SEQ ID No. 1
(b) a DNA capable of hybridizing with the DNA of the above-mentioned (a) under stringent conditions
(c) a DNA having at least (1) 60% identity, (2) 70% identity, (3) 80% identity, (4) 90% identity or (5) 95% identity with the nucleotide sequence shown by nucleotide No. 1065 to 3359 in the nucleotide sequence depicted in SEQ ID No. 1.

9. A gene encoding a protein of the following (a) or (b):
(a) a protein consisting of amino acid number from -1 or 1 to 765 in the amino acid sequence depicted in SEQ ID No. 2
(b) a protein having an amino acid sequence involving deletion, substitution or addition of one to several amino acid(s) in the amino acid sequence (a), which protein has a cyclic lipopeptide acylase activity.

10. A recombinant vector comprising the gene of claim 8 or 9.

11. An expression vector functionally comprising the gene of claim 8 or 9.

12. A transformant obtained by transforming a host cell with a vector of claim 10 or 11.

13. A method of producing cyclic lipopeptide acylase, which comprises
culturing a host cell transformed with the expression vector of claim 11, and
harvesting, from the obtained culture, cyclic lipopeptide acylase capable of catalyzing a reaction to deacylate a side chain acylamino group of a cyclic lipopeptide substance into an amino group.

14. A cyclic lipopeptide acylase produced by the production method of claim 13.

15. A cyclic lipopeptide acylase encoded by a DNA consisting of a nucleotide sequence shown by nucleotide No. 1065 to 3359 in the nucleotide sequence depicted in SEQ ID No. 1.

16. A cyclic lipopeptide acylase which is encoded by a DNA having at least (1) 60% identity, (2) 70% identity, (3) 80% identity, (4) 90% identity or (5) 95% identity with the nucleotide sequence shown by nucleotide No. 1065 to 3359 in the nucleotide sequence depicted in SEQ ID No. 1.

17. A protein of the following (a) or (b):
(a) a protein consisting of amino acid No. -1 to 200 in the amino acid sequence depicted in SEQ ID No. 2
(b) a protein having an amino acid sequence involving deletion, substitution or addition of one to several amino acid(s) in the amino acid sequence (a), which protein forms a complex with the protein of the following (c) or (d) to show a cyclic lipopeptide acylase activity:
(c) a protein consisting of amino acid No. 201 to 765 in the amino acid sequence depicted in SEQ ID No. 2
(d) a protein having an amino acid sequence involving deletion, substitution or addition of one to several amino acid(s) in the amino acid sequence (c), which protein forms a complex with the polypeptide of the above-mentioned (a) or (b) to show a cyclic lipopeptide acylase activity.

18. A protein of the following (c) or (d):
(c) a protein consisting of amino acid No. 201 to 765 in the amino acid sequence depicted in SEQ ID No. 2
(d) a protein having an amino acid sequence involving deletion, substitution or addition of one to several amino acid(s) in the amino acid sequence (c), which protein forms a complex with the protein of (a) or (b) below to show a cyclic lipopeptide acylase activity:
(a) a protein consisting of amino acid number from -1 or 1 to 200 in the amino acid sequence depicted in SEQ ID No. 2
(b) a protein having an amino acid sequence involving deletion, substitution or addition of one to several amino acid(s) in the aminoacid sequence (a), which protein forms a complex with the protein of the above-mentioned (c) or (d) to show a cyclic lipopeptide acylase activity.

19. A DNA encoding the protein of claim 17.

20. A DNA encoding the protein of claim 18.

21. A recombinant vector comprising at least one of claim 19 and claim 20.

22. An expression vector comprising at least one of claim 19 and 20.

23. A transformant obtained by transforming a host cell with the vector of claim 21 or 22.

24. A method of producing cyclic lipopeptide acylase, which comprises
culturing a host cell transformed with the expression vector of claim 22, and
harvesting, from the obtained culture, cyclic lipopeptide acylase capable of catalyzing a reaction to deacylate a side chain acylamino group of a cyclic lipopeptide substance into an amino group.

25. A cyclic lipopeptide acylase produced by the production method of claim 24.

26. A method for deacylating a side chain acylamino group of a cyclic lipopeptide substance into an amino group, which method comprising culturing a host cell transformed with the expression vector of claim 4, 11 or 22, and bringing the cyclic lipopeptide substance into contact with the obtained culture or a treated product thereof.


SEQUENCE LISTING

<110>    Fujisawa Pharmaceutical Co., Ltd.

<120>    A GENE COADING A CYCLIC LOPOPEPTIDE ACYLASE AND AN EXPRESSION
THEREOF

<130>    09368

<150>    JP 11-189644
<151>    1999-7-2

<160>    57

<210>    1
<211>    5692
<212>    DNA
<213>    Streptomyces Sp.
<220>
<221>    CDS
<222>    (948)..(3362)
<400>    1
gaattccgga tggttggaga ggccgatcca gacggtgggc ggggcgaaga ggctgtcggc    60
caggcccgct tcgacgaggt cgaagatcga ggcggcgtcc ggaccgtcca ggatggtgtt    120
ctccgcgccg accgccagat agggcagcag gaacacgtgc atctgggccg agtggtagag    180
cggcagggag tgcacgggcc ggtcggtcgc ggcgaggccg agcgcggtga tcgcgctgac    240
gtactcgtgg accagggccc cgtgcgtcat catcgcgccc ttgggcaggg cggtggtccc    300

ggaggtgtac agcagctgca ccaggtcgtc ggaggcgggc gggcgccgcg gggtgaacgc 360

ccgttccgtc tccagggcgt cgagcagcga gccgggcgcg tcgcggagcg cgcgcaccgg 420

gagtccggcg gggagccgcc cggcgaggtc cgggtcggtc aggacgaggg aggagccgga 480

ctggtcgagg aggtaggcca ggtcgtcgcc ggtgaggttc tggttgaccg gtacgtggac 540

gagaccggcc cgtgcgcagg cgaggaagcc gatcagatag gcgtcggagt tgtgcgcgta 600

ggcggccacc cggtcgccgg gggcgagagc gtactcctcg gtgaggacgg cggcggccgt 660

ggagacggcg gcgtccaggg agcggtaggt ccaggtccgg tcggcgtacc gcacggcggt 720

ccggtcgggg gtgcgccggg cgctgcgggt gaggacgccg tcgactgtgc tgctgcgtac 780

acctgtcatg gcgtgatcct gtgcgtccgg gccctcgggg gtcaagaggc tggataccga 840

ccagacggtt gacagcttcc cgggctccct ggctgagtga cgcttggccg tccgggcgtt 900

ccggaccggc cgcgcccgtg ccacccgtac cgctgggagg aaacacc ttg acg tta 956

Leu Thr Leu

cgc aac cgt ctg aga ctg ctc ggg gtc gcc ggt ctc gcc ctg ttc acc 1004

Arg Asn Arg Leu Arg Leu Leu Gly Val Ala Gly Leu Ala Leu Phe Thr

-35 -30 -25

gtg tcg gcg tcg ctg ccg cct gcc acc gcg tcc ggg acc cag gag acg 1052

Val Ser Ala Ser Leu Pro Pro Ala Thr Ala Ser Gly Thr Gln Glu Thr

-20 -15 -10 -5

cgg cac ccg tcc ggg agc ggt ctt tcg gcc gtc atc cgg tac acg gag 1100

Arg His Pro Ser Gly Ser Gly Leu Ser Ala Val Ile Arg Tyr Thr Glu

1 5 10

tac ggc att ccg cac atc gtg gcg gag gac tac gcg cag ttg ggc ttc 1148

Tyr Gly Ile Pro His Ile Val Ala Glu Asp Tyr Ala Gln Leu Gly Phe

15 20 25

ggc acc ggc tgg gcg cag gcc gcc gat cag gtg tgc acg ctg gcg gac 1196

Gly Thr Gly Trp Ala Gln Ala Ala Asp Gln Val Cys Thr Leu Ala Asp

30 35 40
ggc ttc ctc acg gtg cgc ggg gag cgg tcg agg ttc ttc ggc ccg gac 1244
Gly Phe Leu Thr Val Arg Gly Glu Arg Ser Arg Phe Phe Gly Pro Asp
45 50 55 60
gcc gcc acg gac tac tcc ctc tcc tcg gcg gcg acg aac ctc tcc agc 1292
Ala Ala Thr Asp Tyr Ser Leu Ser Ser Ala Ala Thr Asn Leu Ser Ser
65 70 75
gac ctg tac ttc cgg ggc gtc cgc gac agc ggc acg gtg gag aag ctg 1340
Asp Leu Tyr Phe Arg Gly Val Arg Asp Ser Gly Thr Val Glu Lys Leu
80 85 90
ctc aag gag ccc gcg ccc gcc ggt ccg agc agg gac gtc aag gag acg 1388
Leu Lys Glu Pro Ala Pro Ala Gly Pro Ser Arg Asp Val Lys Glu Thr
95 100 105
atg cgc ggg ttc gcc gcc ggg tac aac gcg tgg atc gcg cag aac cgg 1436
Met Arg Gly Phe Ala Ala Gly Tyr Asn Ala Trp Ile Ala Gln Asn Arg
110 115 120
atc acc gac ccc gcc tgc cgg ggc gcg tcc tgg gtg cgc ccg gtg acg 1484
Ile Thr Asp Pro Ala Cys Arg Gly Ala Ser Trp Val Arg Pro Val Thr
125 130 135 140
gcg ctg gac gtg gcg gcg cgc ggc tac gcg ctg gcg gtg ctc ggc ggc 1532
Ala Leu Asp Val Ala Ala Arg Gly Tyr Ala Leu Ala Val Leu Gly Gly
145 150 155
cag ggg cgc ggc atc gac ggc atc acc gcg gca cag ccg ccg acc gcc 1580
Gln Gly Arg Gly Ile Asp Gly Ile Thr Ala Ala Gln Pro Pro Thr Ala
160 165 170
gct cct ccg gcg gcc ggg gtc acg ccc gag gag gcg gcg acg gcg gcg 1628
Ala Pro Pro Ala Ala Gly Val Thr Pro Glu Glu Ala Ala Thr Ala Ala

175 180 185
gag cgg ctg ctg tcg acg cag aac gcg gac atg ggt tcc aac gcg gtg 1676
Glu Arg Leu Leu Ser Thr Gln Asn Ala Asp Met Gly Ser Asn Ala Val
190 195 200
gcc ttc gac ggc tcc acg acg gtg aac ggg cgc ggg ctg ttg ctc ggc 1724
Ala Phe Asp Gly Ser Thr Thr Val Asn Gly Arg Gly Leu Leu Leu Gly
205 210 215 220
aac ccg cac tac ccg tgg cag ggc gga cgc cgc ttc tgg cag gcg cag 1772
Asn Pro His Tyr Pro Trp Gln Gly Gly Arg Arg Phe Trp Gln Ala Gln
225 230 235
cag acg atc ccc ggc gag ctg aac gtg tcg ggc gcg tcc ctg ctg ggc 1820
Gln Thr Ile Pro Gly Glu Leu Asn Val Ser Gly Ala Ser Leu Leu Gly
240 245 250
gcg acg acg atc tcg atc ggg cac aac gcc gat gtg gcg tgg agc cat 1868
Ala Thr Thr Ile Ser Ile Gly His Asn Ala Asp Val Ala Trp Ser His
255 260 265
acg gtc gcc acg ggc gtc acg ctg aat ctg cat cag ctc agc ctc gat 1916
Thr Val Ala Thr Gly Val Thr Leu Asn Leu His Gln Leu Ser Leu Asp
270 275 280
ccg gcc gac ccg acc gtc tat ctg gtg gac ggg aag cgg gag cgg atg 1964
Pro Ala Asp Pro Thr Val Tyr Leu Val Asp Gly Lys Arg Glu Arg Met
285 290 295 300
acg cag cgg acg gtg agc gtc ccg gtg aag ggc ggg gcc gac gtg acc 2012
Thr Gln Arg Thr Val Ser Val Pro Val Lys Gly Gly Ala Asp Val Thr
305 310 315
cgc acc cag tgg tgg acc cgc tac ggg ccg gtg gcc acc tcg atg ggc 2060
Arg Thr Gln Trp Trp Thr Arg Tyr Gly Pro Val Ala Thr Ser Met Gly

320 325 330
gcg ggg ctg ccg ttg ccg tgg acg gcg agc acg gcg tac gcg ctg aac 2108
Ala Gly Leu Pro Leu Pro Trp Thr Ala Ser Thr Ala Tyr Ala Leu Asn
335 340 345
gat ccg aac gcg acg aat ctg cgg atg gcg gac acc ggt ctg ggc ttc 2156
Asp Pro Asn Ala Thr Asn Leu Arg Met Ala Asp Thr Gly Leu Gly Phe
350 355 360
ggc aag gcc cgc tcc acg ggt gac gtc gag cgt gcg ctg cac cgg tcg 2204
Gly Lys Ala Arg Ser Thr Gly Asp Val Glu Arg Ala Leu His Arg Ser
365 370 375 380
cag ggc atg ccg tgg gtg aac acg atc gcg gcg gac cgg gcg ggt cgc 2252
Gln Gly Met Pro Trp Val Asn Thr Ile Ala Ala Asp Arg Ala Gly Arg
385 390 395
tcg ttc ttc gcg cag tcg cag gtg ctg ccg agg atc acc gac gcg ttg 2300
Ser Phe Phe Ala Gln Ser Gln Val Leu Pro Arg Ile Thr Asp Ala Leu
400 405 410
gcg gag cgc tgc tcg acc ccg ctg ggc cgg gcc acc tac ccc gct tcc 2348
Ala Glu Arg Cys Ser Thr Pro Leu Gly Arg Ala Thr Tyr Pro Ala Ser
415 420 425
ggc ctc gcg gtg ctg gac ggt tcg cgg acg gac tgc gcg ctg ggc agc 2396
Gly Leu Ala Val Leu Asp Gly Ser Arg Thr Asp Cys Ala Leu Gly Ser
430 435 440
gac ccg gac gcg gtg cgg ccg ggg atc ttc ggc ccg ggc cgg atg ccg 2444
Asp Pro Asp Ala Val Arg Pro Gly Ile Phe Gly Pro Gly Arg Met Pro
445 450 455 460
gtg ctg aag aac cag ccg tac gtg gag aac tcc aac gac agc gcg tgg 2492
Val Leu Lys Asn Gln Pro Tyr Val Glu Asn Ser Asn Asp Ser Ala Trp

465 470 475
ctg acc aat gcg gag cgg ccg ctg acc ggg tac gag cgg gtc ttc ggc 2540
Leu Thr Asn Ala Glu Arg Pro Leu Thr Gly Tyr Glu Arg Val Phe Gly
480 485 490
acg atc gcg acg ccc cgg tcg atg cgg acg cgc ggc gcg atc gag gac 2588
Thr Ile Ala Thr Pro Arg Ser Met Arg Thr Arg Gly Ala Ile Glu Asp
495 500 505
gtc gcg tcg atg gcg gac cgg ggc cgc ctc cgg gtc ggg gac ctt cag 2636
Val Ala Ser Met Ala Asp Arg Gly Arg Leu Arg Val Gly Asp Leu Gln
510 515 520
cgg cag cag ttc gcc aac cgt gcg ccg gcc ggg gat ctg gcc gcc tcc 2684
Arg Gln Gln Phe Ala Asn Arg Ala Pro Ala Gly Asp Leu Ala Ala Ser
525 530 535 540
gag gcc gcc aag tgg tgt gcg gcg ctg ccg ggc ggc acg gcc gtg ggc 2732
Glu Ala Ala Lys Trp Cys Ala Ala Leu Pro Gly Gly Thr Ala Val Gly
545 550 555
tcc gac gga acg ccg gtc gac gtg tcg gcg gcc tgc cgg gtg ctg cgg 2780
Ser Asp Gly Thr Pro Val Asp Val Ser Ala Ala Cys Arg Val Leu Arg
560 565 570
cgc tgg gac cgg acc gtg gac agc gac agc cgg ggc gcg ctg ctc ttc 2828
Arg Trp Asp Arg Thr Val Asp Ser Asp Ser Arg Gly Ala Leu Leu Phe
575 580 585
gac cgg ttc tgg cgg aag gcg tcg tcg gcg ccc gcc gcc gag ctg tgg 2876
Asp Arg Phe Trp Arg Lys Ala Ser Ser Ala Pro Ala Ala Glu Leu Trp
590 595 600
agg acg ccg ttc gat ccg gcc gac ccg gtg cgc act ccg cgc ggc ctg 2924
Arg Thr Pro Phe Asp Pro Ala Asp Pro Val Arg Thr Pro Arg Gly Leu

605                 610                 615                 620

aac acg gcc gcg ccc gtc ctg ggc agg gcc ctg gcg gac gcc gtg gcg     2962

Asn Thr Ala Ala Pro Val Leu Gly Arg Ala Leu Ala Asp Ala Val Ala

                625                 630                 635

gag ctg cgg gcg gcg ggc atc gcg ctg gac gcc ccg ctg ggc gag cac     3020

Glu Leu Arg Ala Ala Gly Ile Ala Leu Asp Ala Pro Leu Gly Glu His

            640                 645                 650

cag ttc gtc gtg cgg aac ggc aag cgg ctc ccg atc ggc ggc ggg acg     3068

Gln Phe Val Val Arg Asn Gly Lys Arg Leu Pro Ile Gly Gly Gly Thr

        655                 660                 665

gag tcg ctg ggc atc tgg aac aag acc gag ccg cag tgg aac gcg gcg     3116

Glu Ser Leu Gly Ile Trp Asn Lys Thr Glu Pro Gln Trp Asn Ala Ala

    670                 675                 680

ggc ggc ggc tat acg gag gtg tcg tcg ggc tcc agc tac atc cag gcg     3164

Gly Gly Gly Tyr Thr Glu Val Ser Ser Gly Ser Ser Tyr Ile Gln Ala

685                 690                 695                 700

gtc ggc tgg gac gac agc cgc tgc ccg gtg gcc cgg acg ctg ctg acg     3212

Val Gly Trp Asp Asp Ser Arg Cys Pro Val Ala Arg Thr Leu Leu Thr

                705                 710                 715

tac tcc cag tcg gag aac ccg aag tca ccg cac tac agc gac cag acc     3260

Tyr Ser Gln Ser Glu Asn Pro Lys Ser Pro His Tyr Ser Asp Gln Thr

            720                 725                 730

agg ctg tac gcg ggt gag cgc tgg gtg acg tcc cgg ttc tgc gag agg     3308

Arg Leu Tyr Ala Gly Glu Arg Trp Val Thr Ser Arg Phe Cys Glu Arg

        735                 740                 745

gac atc gcg cgt tcg ccg gac ctg cgg gtg gtg cgg gtg cac gag cgg     3356

Asp Ile Ala Arg Ser Pro Asp Leu Arg Val Val Arg Val His Glu Arg

750                 755                 760

cgg tag cgcggtg ggcggacggg cccgcccatc cgcggcgaga agggcgtccg          3409

Arg

765

cctcggcggg cgcccttctc accgatgtgt cgtgaccgcg ctcccggggg cgtcctcacc   3469

gagccgccga agggcccggc ggccgaaccc gtgaccatgc gtgcgacgca tcacgctccg   3529

tcggctccgc cctccgcccg cgcccaggcc agctgcgcgt cgctcagcgg cgggtcgaag   3589

ccttccggga acagcagcat ccgcggctgc ggccacatgt tctccggtcc gtgttcctga   3649

cagtccaggg cgaggagatg cggcccgtcc ccgcaggact cgtgccggta ggggcggtcg   3709

tgcgcccggc agaaatagcc gaacaccgca cagtggtcgt cgccgcccgg tcggtggaag   3769

ccggggtcgc tgacgatcac ggtcaccggc tcctgccggt tgagccgagg gatgggccgg   3829

ggatcacgcc acaacagtcg aggagggagc acacgctcat cttccccggg gccgagccca   3889

cgggaagggg gagcacggcg ggacgcctcc cgtcggcgtg atcgaccggg ccgtcccgct   3949

cgcgggcggg ccctcccgga cccgttgctc tacagcgggc gctcgaagcc ctcccagtac   4009

ggttcgcgca gccgccgttt gtagagcttg ccgttggggt cgcggggcat ggcggtgatg   4069

aagtcgaggc tccggggtcg tttgtagccg gcgagccgct cctcgcagtg ggcgaggatc   4129

gcggcggcga gcgcgggtga cggctcgtgg ccatcggccg gttcgacgac ggccttgacc   4189

tcctcgccgc ggtcggcgtg ggggatgccg aaggcggcgg cgtccgcgac ggcggggtgg   4249

gtgagcagga ccgactcgat ctcggcgggg tagatgttga ccccgcccgc gatgatcatg   4309

tcgatcttgc ggtcgcggag gaagaggtag ccgtcctcgt ccagcacgcc gaggtcaccg   4369

acggtgaaga agtcgccgat gcggttcgtg cgggtcttgg tctcgtcctt gtggtagctg   4429

aagccgccgg tgctcatctt catgtagacg gtgcccagtt cgcctggcgg gaggcggttg   4489

ccgtcgtcgt cgaagacggc cagttcgctg atcggccagg ccttgccgac ggtgccgggc   4549

ttcttcagcc agtcctcggc ggtggcgaac gctcccccgc cctcgctggc cgcgtagtac   4609

tcctcgacgc agctccccca ccagtcgatc atggcgcgtt tgacgtggtc ggggcagggg   4669

gctgccccgt ggatggcgtg ccgcatggag gagacgtcgt agcgggacct cacctcgtcg   4729

ggcagcgcga gcagccggtg gaactgggtg gggaccatgt gggtgtgggt gcagcggtgg   4789

gcgtcgacga ggcgcagcat ctcctcgggc gaccagccgt ccatcaggac cagcgggtgg 4849
ccgatgtgca gggcggcgcc cgcgaattgg agtacggcgg tgtggtagag cggcgagcag 4909
accaggtgga cgttgtcgtc gaacggccgg atgccgaaga tgccgaggaa cccgccgagg 4969
taggtctcct cggggcgttt gccgggcagg gggcgccgga tgccgcgggg gcggccggtg 5029
gtgcccgagg tgtagttcat gacccagccg agggtgcggt tctcaggcgg cgtggcgggg 5089
tggccttcga ggagttcggc ccaggggcgg cagccgggga ccgtgccgac gccgtagcgg 5149
tgggtcgcgg gcagttccgc ctcgtcggcg gcggccgtcg cggtggccgc gaagcgttcg 5209
tgggcgatca ggacgcgggc gccggagtcg gcgacgatcc aggcgatctc ggggccgacg 5269
aggtggtggt tgaccggcac gaggtagaag ccggcctgcg aggcggcgag gtgggcggtg 5329
aggagttcga cgccgttggg caggacgacg gcgaacgcgt cgccctcgcg cagtccggcc 5389
gcgcgcaggc cgtggaccat gcggttgacg tcggcgtgca ggcggcccgc gctccactcc 5449
tcgccgtcgg gggcgatcag gacggtgcgg tcggggtcgg ctgcggcctg ggcccagaaa 5509
ccgttgggcg gctggttcac gtggcactcc ttccggcgat gcggttcatg cgggtgacgg 5569
cccgttcgaa gccgcgggtc aggtcgtcga cgacggcccg gacgctgcgt tcactggtca 5629
tccggccgac gatctgcccg acgggcgtgc cgagcagctc gccgacctcg tacttctgga 5689
tcc 5692

<210> 2
<211> 765
<212> PRT
<213> Streptomyces Sp.
<400> 2
Leu Thr Leu Arg Asn Arg Leu Arg Leu Leu Gly Val Ala Gly Leu Ala
-35 -30 -25
Leu Phe Thr Val Ser Ala Ser Leu Pro Pro Ala Thr Ala Ser Gly Thr
-20 -15 -10
Gln Glu Thr Arg His Pro Ser Gly Ser Gly Leu Ser Ala Val Ile Arg

-5                    1                    5
Tyr Thr Glu Tyr Gly Ile Pro His Ile Val Ala Glu Asp Tyr Ala Gln
10                    15                    20                    25
Leu Gly Phe Gly Thr Gly Trp Ala Gln Ala Ala Asp Gln Val Cys Thr
                    30                    35                    40
Leu Ala Asp Gly Phe Leu Thr Val Arg Gly Glu Arg Ser Arg Phe Phe
                45                    50                    55
Gly Pro Asp Ala Ala Thr Asp Tyr Ser Leu Ser Ser Ala Ala Thr Asn
            60                    65                    70
Leu Ser Ser Asp Leu Tyr Phe Arg Gly Val Arg Asp Ser Gly Thr Val
        75                    80                    85
Glu Lys Leu Leu Lys Glu Pro Ala Pro Ala Gly Pro Ser Arg Asp Val
90                    95                    100                    105
Lys Glu Thr Met Arg Gly Phe Ala Ala Gly Tyr Asn Ala Trp Ile Ala
                    110                    115                    120
Gln Asn Arg Ile Thr Asp Pro Ala Cys Arg Gly Ala Ser Trp Val Arg
                125                    130                    135
Pro Val Thr Ala Leu Asp Val Ala Ala Arg Gly Tyr Ala Leu Ala Val
            140                    145                    150
Leu Gly Gly Gln Gly Arg Gly Ile Asp Gly Ile Thr Ala Ala Gln Pro
        155                    160                    165
Pro Thr Ala Ala Pro Pro Ala Ala Gly Val Thr Pro Glu Glu Ala Ala
170                    175                    180                    185
Thr Ala Ala Glu Arg Leu Leu Ser Thr Gln Asn Ala Asp Met Gly Ser
                    190                    195                    200
Asn Ala Val Ala Phe Asp Gly Ser Thr Thr Val Asn Gly Arg Gly Leu
                205                    210                    215

Leu Leu Gly Asn Pro His Tyr Pro Trp Gln Gly Gly Arg Arg Phe Trp
220 225 230
Gln Ala Gln Gln Thr Ile Pro Gly Glu Leu Asn Val Ser Gly Ala Ser
235 240 245
Leu Leu Gly Ala Thr Thr Ile Ser Ile Gly His Asn Ala Asp Val Ala
250 255 260 265
Trp Ser His Thr Val Ala Thr Gly Val Thr Leu Asn Leu His Gln Leu
270 275 280
Ser Leu Asp Pro Ala Asp Pro Thr Val Tyr Leu Val Asp Gly Lys Arg
285 290 295
Glu Arg Met Thr Gln Arg Thr Val Ser Val Pro Val Lys Gly Gly Ala
300 305 310
Asp Val Thr Arg Thr Gln Trp Trp Thr Arg Tyr Gly Pro Val Ala Thr
315 320 325
Ser Met Gly Ala Gly Leu Pro Leu Pro Trp Thr Ala Ser Thr Ala Tyr
330 335 340 345
Ala Leu Asn Asp Pro Asn Ala Thr Asn Leu Arg Met Ala Asp Thr Gly
350 355 360
Leu Gly Phe Gly Lys Ala Arg Ser Thr Gly Asp Val Glu Arg Ala Leu
365 370 375
His Arg Ser Gln Gly Met Pro Trp Val Asn Thr Ile Ala Ala Asp Arg
380 385 390
Ala Gly Arg Ser Phe Phe Ala Gln Ser Gln Val Leu Pro Arg Ile Thr
395 400 405
Asp Ala Leu Ala Glu Arg Cys Ser Thr Pro Leu Gly Arg Ala Thr Tyr
410 415 420 425
Pro Ala Ser Gly Leu Ala Val Leu Asp Gly Ser Arg Thr Asp Cys Ala

430 435 440
Leu Gly Ser Asp Pro Asp Ala Val Arg Pro Gly Ile Phe Gly Pro Gly
445 450 455
Arg Met Pro Val Leu Lys Asn Gln Pro Tyr Val Glu Asn Ser Asn Asp
460 465 470
Ser Ala Trp Leu Thr Asn Ala Glu Arg Pro Leu Thr Gly Tyr Glu Arg
475 480 485
Val Phe Gly Thr Ile Ala Thr Pro Arg Ser Met Arg Thr Arg Gly Ala
490 495 500 505
Ile Glu Asp Val Ala Ser Met Ala Asp Arg Gly Arg Leu Arg Val Gly
510 515 520
Asp Leu Gln Arg Gln Gln Phe Ala Asn Arg Ala Pro Ala Gly Asp Leu
525 530 535
Ala Ala Ser Glu Ala Ala Lys Trp Cys Ala Ala Leu Pro Gly Gly Thr
540 545 550
Ala Val Gly Ser Asp Gly Thr Pro Val Asp Val Ser Ala Ala Cys Arg
555 560 565
Val Leu Arg Arg Trp Asp Arg Thr Val Asp Ser Asp Ser Arg Gly Ala
570 575 580 585
Leu Leu Phe Asp Arg Phe Trp Arg Lys Ala Ser Ser Ala Pro Ala Ala
590 595 600
Glu Leu Trp Arg Thr Pro Phe Asp Pro Ala Asp Pro Val Arg Thr Pro
605 610 615
Arg Gly Leu Asn Thr Ala Ala Pro Val Leu Gly Arg Ala Leu Ala Asp
620 625 630
Ala Val Ala Glu Leu Arg Ala Ala Gly Ile Ala Leu Asp Ala Pro Leu
635 640 645

Gly Glu His Gln Phe Val Val Arg Asn Gly Lys Arg Leu Pro Ile Gly
650                 655                 660                 665
Gly Gly Thr Glu Ser Leu Gly Ile Trp Asn Lys Thr Glu Pro Gln Trp
                670                 675                 680
Asn Ala Ala Gly Gly Gly Tyr Thr Glu Val Ser Ser Gly Ser Ser Tyr
            685                 690                 695
Ile Gln Ala Val Gly Trp Asp Asp Ser Arg Cys Pro Val Ala Arg Thr
        700                 705                 710
Leu Leu Thr Tyr Ser Gln Ser Glu Asn Pro Lys Ser Pro His Tyr Ser
    715                 720                 725
Asp Gln Thr Arg Leu Tyr Ala Gly Glu Arg Trp Val Thr Ser Arg Phe
730                 735                 740                 745
Cys Glu Arg Asp Ile Ala Arg Ser Pro Asp Leu Arg Val Val Arg Val
                750                 755                 760
His Glu Arg Arg
            765
<210>    3
<211>    20
<212>    PRT
<213>    Streptomyces Sp.
<400>    3
Ser Asn Ala Val Ala Phe Asp Gly Ser Thr Thr Val Asn Gly Arg Gly
 1               5                  10                  15
Leu Leu Leu Gly
            20

<210>    4

<211>    20

<212>    PRT

<213>    Streptomyces Sp.

<400>    4

Gly Ser Gly Leu Ser Ala Val Ile Arg Tyr Thr Glu Tyr Gly Ile Pro

1               5                   10                  15

His Ile Val Ala

            20

<210>    5

<211>    20

<212>    PRT

<213>    Streptomyces Sp.

<400>    5

Gly Ser Gly Leu Ser Ala Val Ile Arg Tyr Thr Glu Tyr Gly Ile Pro

1               5                   10                  15

His His Val Ala

            20

<210>    6

<211>    15

<212>    DNA

<213>    Artificial Sequence

<220>

<223>    Oligonucleotide designed to act as PCR primer (forward) to

amplify the DNA coding N-terminal amino acid sequences of FR901379

acyrase small subunit.

<400>    6

ctstcsgcsg tsatc                                                    15

<210>    7

<211>    15

<212>    DNA

<213>    Artificial Sequence

<220>

<223>    Oligonucleotide designed to act as PCR primer (reverse) to

amplify the DNA coding N-terminal amino acid sequences of FR901379

acyrase small subunit.

<400>    7

gtggtgsggg atscc                                                    15

<210>    8

<211>    16

<212>    DNA

<213>    Artificial Sequence

<220>

<223>    Oligonucleotide designed to act as PCR primer (forward) to

amplify the DNA coding N-terminal amino acid sequences of FR901379

acyrase large subunit.

<400>    8

csgtsgcstt cgacgg                                                   16

<210>    9

<211>    15

<212>	DNA
<213>	Artificial Sequence
<220>
<223>	Oligonucleotide designed to act as PCR primer (reverse) to
amplify the DNA coding N-terminal amino acid sequences of FR901379
acyrase large subunit.
<400>	9
sccsagsags agscc	15

<210>	10
<211>	20
<212>	DNA
<213>	Artificial Sequence
<220>
<223>	Oligonucleotide designed to act as PCR primer (forward) to
amplify the DNA coding sequence between FR901379 acyrase small subunit
and large subunit.
<400>	10
atccggtaca cggagtacgg	20

<210>	11
<211>	19
<212>	DNA
<213>	Artificial Sequence
<220>
<223>	Oligonucleotide designed to act as PCR primer (reverse) to
amplify the DNA coding sequence between FR901379 acyrase small subunit

and large subunit.
<400>	11
cgttcaccgt cgtggagcc	19

<210>	12
<211>	10
<212>	DNA
<213>	Artificial Sequence
<220>
<223>	Oligonucleotide designed for use in changing site from EcoR I
site to Sac I site.
<400>	12
aattgagctc	10

<210>	13
<211>	16
<212>	DNA
<213>	Artificial Sequence
<220>
<223>	Oligonucleotide designed to act as sequencing primer.
<400>	13
caactgcgcg tagtcc	16

<210>	14
<211>	16
<212>	DNA
<213>	Artificial Sequence

<220>

<223>	Oligonucleotide designed to act as sequencing primer.

<400>	14

catgggttcc aacgcg	16

<210>	15

<211>	16

<212>	DNA

<213>	Artificial Sequence

<220>

<223>	Oligonucleotide designed to act as sequencing primer.

<400>	15

gctgtcaacc gtctgg	16

<210>	16

<211>	16

<212>	DNA

<213>	Artificial Sequence

<220>

<223>	Oligonucleotide designed to act as sequencing primer.

<400>	16

acgcgctgaa cgatcc	16

<210>	17

<211>	16

<212>	DNA

<213>	Artificial Sequence

<220>

<223>	Oligonucleotide designed to act as sequencing primer.

<400>	17

cggacctgga cctacc	16

<210>	18

<211>	17

<212>	DNA

<213>	Artificial Sequence

<220>

<223>	Oligonucleotide designed to act as sequencing primer.

<400>	18

gtgggtgaac acgatcg	17

<210>	19

<211>	16

<212>	DNA

<213>	Artificial Sequence

<220>

<223>	Oligonucleotide designed to act as sequencing primer.

<400>	19

gaccttcagc ggcagc	16

<210>	20

<211>	16

<212>	DNA

<213>	Artificial Sequence

<220>

<223>	Oligonucleotide designed to act as sequencing primer.

<400>	20

caagtggtgt gcggcg	16

<210>	21

<211>	16

<212>	DNA

<213>	Artificial Sequence

<220>

<223>	Oligonucleotide designed to act as sequencing primer.

<400>	21

gtcgctgggc atctgg	16

<210>	22

<211>	16

<212>	DNA

<213>	Artificial Sequence

<220>

<223>	Oligonucleotide designed to act as sequencing primer.

<400>	22

gctgctgacg tactcc	16

<210>	23

<211>	16

<212>	DNA

<213>	Artificial Sequence

<220>

<223>	Oligonucleotide designed to act as sequencing primer.

<400>	23

gtcaaccgca tggtcc	16

<210>	24

<211>	16

<212>	DNA

<213>	Artificial Sequence

<220>

<223>	Oligonucleotide designed to act as sequencing primer.

<400>	24

atcgcctgga tcgtcg	16

<210>	25

<211>	16

<212>	DNA

<213>	Artificial Sequence

<220>

<223>	Oligonucleotide designed to act as sequencing primer.

<400>	25

cgtcagcgcg atcacc	16

<210>	26

<211>	16

<212>	DNA

<213>	Artificial Sequence

<220>

<223>	Oligonucleotide designed to act as sequencing primer.

<400>	26

ggtgtacagc agctgc	16

<210>	27

<211>	16

<212>	DNA

<213>	Artificial Sequence

<220>

<223>	Oligonucleotide designed to act as sequencing primer.

<400>	27

ctccctcgtc ctgacc	16

<210>	28

<211>	16

<212>	DNA

<213>	Artificial Sequence

<220>

<223>	Oligonucleotide designed to act as sequencing primer.

<400>	28

gagttgtgcg cgtagg	16

<210>	29

<211>	16

<212>	DNA

<213>	Artificial Sequence

<220>

<223>	Oligonucleotide designed to act as sequencing primer.

<400>	29

tgacgcttgg ccgtcc	16

<210>	30

<211>	16

<212>	DNA

<213>	Artificial Sequence

<220>

<223>	Oligonucleotide designed to act as sequencing primer.

<400>	30

gactacgcgc agttgg	16

<210>	31

<211>	16

<212>	DNA

<213>	Artificial Sequence

<220>

<223>	Oligonucleotide designed to act as sequencing primer.

<400>	31

tacaacgcgt ggatcg	16

<210>	32

<211>	16

<212>	DNA

<213>	Artificial Sequence

<220>

<223>	Oligonucleotide designed to act as sequencing primer.

<400>	32

ggtgatccgg ttctgc	16

<210>	33

<211>	16

<212>	DNA

<213>	Artificial Sequence

<220>

<223>	Oligonucleotide designed to act as sequencing primer.

<400>	33

gggtagtgcg ggttgc	16

<210>	34

<211>	16

<212>	DNA

<213>	Artificial Sequence

<220>

<223>	Oligonucleotide designed to act as sequencing primer.

<400>	34

ctgcatcagc tcagcc	16

<210>	35

<211>	16

<212>	DNA

<213>	Artificial Sequence

<220>

<223>	Oligonucleotide designed to act as sequencing primer.

<400>	35

gtccaccact gggtgc	16

<210>	36

<211>	16

<212>	DNA

<213>	Artificial Sequence

<220>

<223>	Oligonucleotide designed to act as sequencing primer.

<400>	36

gaagcggggt aggtgg	16

<210>	37

<211>	16

<212>	DNA

<213>	Artificial Sequence

<220>

<223>	Oligonucleotide designed to act as sequencing primer.

<400>	37

ccggtgctga agaacc	16

<210>	38

<211>	16

<212>	DNA

<213>	Artificial Sequence

<220>

<223>	Oligonucleotide designed to act as sequencing primer.

<400>	38

ctgccgctga aggtcc	16

<210>	39

<211>	16

<212>	DNA

<213>	Artificial Sequence

<220>

<223>	Oligonucleotide designed to act as sequencing primer.

<400>	39

tcgaacggcg tcctcc	16

<210>	40

<211>	16

<212>	DNA

<213>	Artificial Sequence

<220>

<223>	Oligonucleotide designed to act as sequencing primer.

<400>	40

tggaggacgc cgttcg	16

<210>	41

<211>	16

<212>	DNA

<213>	Artificial Sequence

<220>

<223>	Oligonucleotide designed to act as sequencing primer.

<400>	41

gcctggatgt agctgg	16

<210>	42

<211>	16

<212>	DNA

<213>	Artificial Sequence

<220>

<223>	Oligonucleotide designed to act as sequencing primer.

<400>	42

ggacatcgcg cgttcg	16

<210>	43

<211>	16

<212>	DNA

<213>	Artificial Sequence

<220>

<223>	Oligonucleotide designed to act as sequencing primer.

<400>	43

cgaacgcgcg atgtcc	16

<210>	44

<211>	16

<212>	DNA

<213>	Artificial Sequence

<220>

<223>	Oligonucleotide designed to act as sequencing primer.

<400>	44

ccgtgaccat gcgtgc	16

<210>	45

<211>	16

<212>	DNA

<213>	Artificial Sequence

<220>

<223>	Oligonucleotide designed to act as sequencing primer.

<400>	45

gcacgcatgg tcacgg	16

<210>	46

<211>	16

<212>	DNA

<213>	Artificial Sequence

<220>

<223>	Oligonucleotide designed to act as sequencing primer.

<400>	46

gaggagacct acctcg	16

<210>	47

<211>	16

<212>	DNA

<213>	Artificial Sequence

<220>

<223>	Oligonucleotide designed to act as sequencing primer.

<400>	47

aggtcccgct acgacg	16

<210>	48

<211>	16

<212>	DNA

<213>	Artificial Sequence

<220>

<223>	Oligonucleotide designed to act as sequencing primer.

<400>	48

gaccatgcgg ttgacg	16

<210>	49

<211>	16

<212>	DNA

<213>	Artificial Sequence

<220>

<223>	Oligonucleotide designed to act as sequencing primer.

<400>	49

cagttccgcc tcgtcg	16

<210>	50

<211>	16

<212>	DNA

<213>	Artificial Sequence

<220>

<223>	Oligonucleotide designed to act as sequencing primer.

<400>	50

caggtggacg ttgtcg	16

<210>	51

<211>	16

<212>	DNA

<213>	Artificial Sequence

<220>

<223>	Oligonucleotide designed to act as sequencing primer.

<400>	51

gtcgctgacg atcacg	16

<210>	52

<211>	16

<212>	DNA

<213>	Artificial Sequence

<220>

<223>	Oligonucleotide designed to act as sequencing primer.

<400>	52

gtgatcgtca gcgacc	16

<210>	53

<211>	16

<212>	DNA

<213>	Artificial Sequence

<220>

<223>	Oligonucleotide designed to act as sequencing primer.

<400>	53

ggcggtgatg aagtcg	16

<210>	54

<211>	16

<212>	DNA

<213>	Artificial Sequence

<220>

<223>	Oligonucleotide designed to act as sequencing primer.

<400>	54

cgacttcatc accgcc	16

<210>	55

<211>	16

<212>	DNA

<213>	Artificial Sequence

<220>

<223>	Oligonucleotide designed to act as sequencing primer.

<400>	55

ggcgacttct tcaccg	16

<210>	56

<211>	16

<212>	DNA

<213>	Artificial Sequence

<220>

<223>	Oligonucleotide designed to act as sequencing primer.

<400>	56

cggtgaagaa gtcgcc	16

<210>	57

<211>	16

<212>	DNA

<213>	Artificial Sequence

<220>

<223>	Oligonucleotide designed to act as sequencing primer.

<400>	57

ccagacggtt gacagc	16